# FASCICULE DE BREVET EUROPEEN

(11) **EP 1 200 391 B1**
(45) Date de publication et mention de la délivrance du brevet: **19.10.2005**
(21) Numéro de dépôt: 00958670.2
(22) Date de dépôt: 11.08.2000
(51) Int. Cl.: C07C 235/04, C07C 235/06, C02F 1/26, C07D 295/18

(54) **DERIVES ACETAMIDO DE CALIXARENES, LEUR PREPARATION ET LEUR UTILISATION POUR L'EXTRACTION DU STRONTIUM**
ACETAMIDOCALIXARENDERIVATE, IHRE HERSTELLUNG SOWIE IHRE ANWENDUNG FÜR DIE EXTRAKTION VON STRONTIUM
CALIXARENE ACETAMIDO DERIVATIVES, PREPARATION AND USE THEREOF FOR EXTRACTING STRONTIUM

(30) Priorité: 13.08.1999 FR 9910480
(43) Date de publication de la demande: 02.05.2002
(73) Titulaire: COMMISSARIAT A L'ENERGIE ATOMIQUE, 75752 Paris Cédex 15 (FR)
(72) Inventeur: DOZOL, Jean-François, F-04660 Pierrevert (FR); UNGARO, Rocco, I-43100 Parme (IT); CASNATI, Alessandro, I-43100 Parme (IT)
(74) Mandataire: Audier, Philippe André
(86) Numéro de dépôt international: PCT/FR2000/002304
(87) Numéro de publication internationale: WO 2001/012586

(56) Documents cités:
- EP-A- 0 309 291
- EP-A- 0 432 989
- WO-A-94/12502
- WO-A-94/24138
- WO-A-97/17322
- SUK-KYU CHANG ET AL.: "Calixarene-based amide ionophores for IIA metal cations" CHEMISTRY LETTERS., 1987, pages 947-948, XP000891490 CHEMICAL SOCIETY OF JAPAN. TOKYO., JP ISSN: 0366-7022
- FRANÇOISE ARNAUD-NEU ET AL.: "Cation complexation by chemically modified calixarenes." JOURNAL OF THE CHEMICAL SOCIETY, PERKIN TRANSACTIONS 2., 1997, pages 575-579, XP002133937 LETCHWORTH GB
- PATENT ABSTRACTS OF JAPAN vol. 1996, no. 09, 30 septembre 1996 (1996-09-30) & JP 08 127561 A (FUKUOKA PREF GOV), 21 mai 1996 (1996-05-21)
- TAKESHI NAGASAKI ET AL.: "Synthesis and solvent extraction studies of novel calixarene-based uranophiles bearing hydroxamic groups" JOURNAL OF THE CHEMICAL SOCIETY, PERKIN TRANSACTIONS 2., 1991, pages 1063-101066, XP002133938 LETCHWORTH GB
- ANDREETTI G.D. ET AL: 'SOLID STATE STUDIES on p. t-BUTYL-CALIX[6]ARENE DERIVATIVES' J. INCLUSION PHENOM. vol. 5, 1987, pages 123 - 126

## Description

### Domaine technique

La présente invention a pour objet de nouveaux dérivés de calixarènes portant des groupements amides, leur procédé de préparation et leur utilisation pour l'extraction du strontium.

Ces nouveaux dérivés de calixarènes peuvent être utilisés pour l'extraction du strontium présent dans des solutions aqueuses provenant d'installations de retraitement de combustibles nucléaires usés.

### État de la technique antérieure

On a déjà envisagé d'utiliser des ligands macrocycliques tels que les calixarènes, pour extraire des métaux à partir de solutions aqueuses. Ainsi, le document EP 0 432 989 propose des calixarènes et oxacalixarènes pour l'extraction de métaux en général, et de métaux lourds ou précieux en particulier, tandis que les documents WO94/12502 [1] et WO94/24138 [2] décrivent des calixarènes-couronnes utilisables pour l'extraction sélective du césium et des actinides.

L'extraction du césium à partir de solutions aqueuses est intéressante car le césium donne un dégagement thermique qui gêne l'entreposage de solutions nitriques de produits de fission.

Le strontium 90 est également un isotope donnant un dégagement thermique important, le strontium et le césium contribuent à plus de 90% au dégagement thermique des solutions de produits de fission.

Aussi, leur élimination de ces solutions faciliterait ainsi l'entreposage de ces dernières. Par ailleurs, l'élimination du césium, du strontium et des actinides peut permettre de déclassifier des déchets de type B et de les adresser à un stockage de surface existant plutôt qu'à un site d'entreposage ou de stockage en formation géologique.

Des solutions de ce type peuvent être par exemple des concentrats d'évaporateur ou des solutions générées par les opérations de démantèlement.

La présente invention a précisément pour objet de nouveaux dérivés de calixarènes

### Exposé de l'invention

La présente invention a précisément pour objet de nouveaux dérivés de calixarènes qui conduisent à une extraction satisfaisante du strontium.

Selon l'invention, le nouveau dérivé de calixarène répond à la formule : 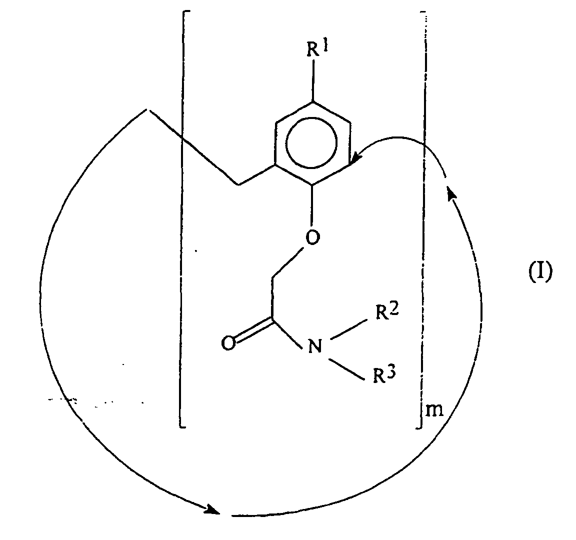 dans laquelle :
- R¹ représente un groupe hydroxyle, alcoxy, aryloxy, arylalcoxy, ou cycloalcoxy, ou un groupe de formule O(CH₂)ₙ[O(CH₂)ₚ]_{q} OR⁴ (II) dans laquelle R⁴ représente un atome d'hydrogène ou un groupe alkyle, n et p sont des nombres entiers allant de 1 à 6, et q est égal à 0 ou est un nombre entier de 1 à 6,
- R² et R³ qui peuvent être identiques ou différents, représentent un groupe alkyle, cycloalkyle ou aryle, ou un groupe de formule : O(CH₂)ₙ [O(CH₂)ₚ]_{q} OR⁴ (II) dans laquelle R⁴, n, p et q sont tels que définis ci-dessus, ou
- R² et R³ forment avec l'atome d'azote auquel ils sont liés un groupe hétérocyclique choisi parmi les groupes pipéridyle, pyrrolidinyle, morpholinyle, thiomorpholinyle, tétrahydropyridyle, imidazolidinyle, indolinyle, tétrahydroquinolyle et perhydroindolyle, et
- m est un nombre entier allant de 6 à 8.

Dans cette formule, les groupes alkyle et alcoxy peuvent être des groupes linéaires ou ramifiés, ayant de préférence de 1 à 12 atomes de carbone. Les groupes aryle et aryloxy utilisables sont des groupes monovalents dérivés d'un noyau aromatique ou hétérocyclique par enlèvement d'un atome d'hydrogène à l'un des atomes de carbone du cycle.

A titre d'exemple de tels groupes, on peut citer les groupes phényle, naphtyle, pyridyle, thiophényle, et phényle substitué.

On peut aussi utiliser pour R¹ des groupes arylalcoxy dans lesquels le groupe alcoxy a de préférence de 1 à 12 atomes de carbone.

A titre d'exemple de tels groupes, on peut citer le groupe benzyloxy et le groupe benzyle.

Lorsque R¹ représente le groupe de formule (II), R⁴ peut être un atome d'hydrogène ou un groupe alkyle ayant de préférence de 1 à 12 atomes de carbone.

Selon l'invention, R¹ est de préférence un groupe alcoxy ou arylalcoxy, par exemple le groupe pentoxy ou octoxy, ou le groupe benzyloxy.

Selon l'invention, R² et R³ peuvent être des groupes alkyle, cycloalkyle ou aryle ayant de 1 à 12 atomes de carbone.

De préférence R² et R³ sont des groupes alkyle, par exemple le groupe éthyle.

Les calixarènes peuvent comprendre de 6 à 8 cycles phényle et de préférence le dérivé comprend 6 ou 8 cycles phényle, c'est-à-dire que m est égal à 6 ou 8.

Les dérivés de calixarène de l'invention peuvent être préparés par des procédés faciles à mettre en oeuvre.

Ainsi, lorsque R¹ représente un groupe benzyloxy, on peut préparer le calixarène de formule (I) dans laquelle R² et R³ sont tels que définis ci-dessus, par un procédé comprenant la réaction d'un calixarène de formule : 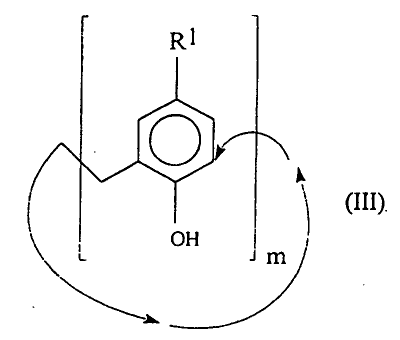 dans laquelle R¹ et m sont tels que définis ci-dessus avec un chloroacétamide de formule : 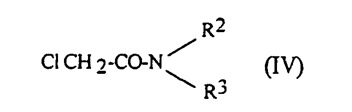 dans laquelle R² et R³ sont tels que définis ci-dessus.

Lorsque dans le calixarène de formule (I) décrit ci-dessus, R¹ représente un groupe alcoxy, on peut préparer ce dérivé de calixarène par un procédé comprenant les étapes suivantes :
a) la réaction d'un calixarène de formule : 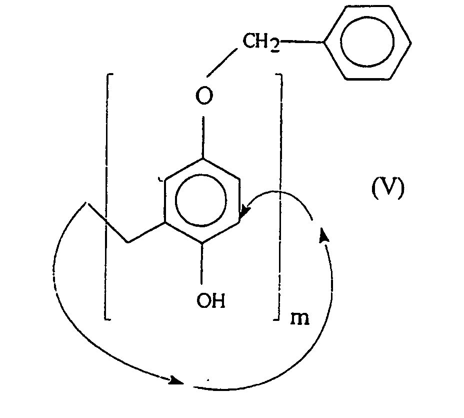 dans laquelle m est tel que défini ci-dessus, avec un chloroacétamide de formule : 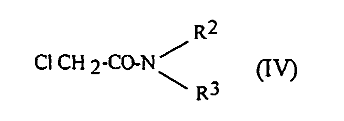 dans laquelle R² et R³ sont tels que définis ci-dessus, pour obtenir le dérivé de formule : 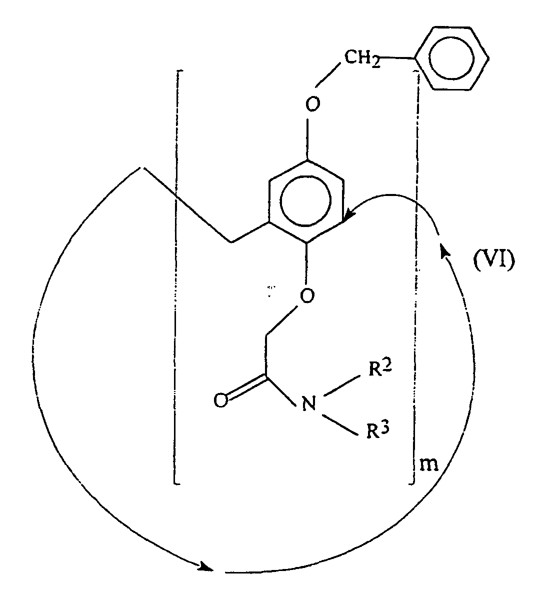 dans laquelle R² et R³ sont tels que définis ci-dessus,
b) réaction du dérivé de formule (VI) avec Pd(OH₂) pour obtenir le calixarène de formule : 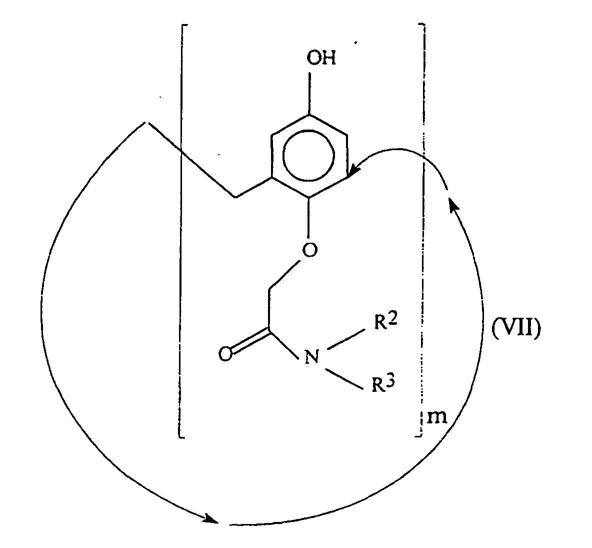
c) réaction du calixarène de formule VII avec un dérivé halogéné de formule R⁵X dans laquelle R⁵ représente un groupe alkyle et X est un atome d'halogène pour obtenir le dérivé de calixarène de formule (I) dans laquelle R¹ représente le groupe alcoxy OR⁵.

Les calixarènes de formule (III) utilisés comme produits de départ pour la préparation des dérivés de l'invention peuvent être préparés par réaction de condension du p-benzyloxyphénol de formule : 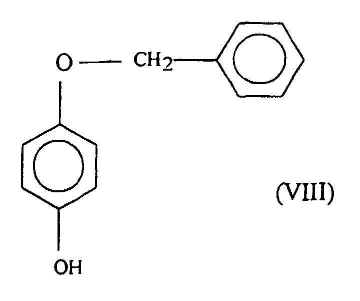 pour obtenir un mélange des calix[6]arène, calix[7]arène et calix[8]arène de formule (III), et séparation du calixarène de formule (III) avec n = 6 ou 8.

Les calixarènes de formule (I) de l'invention peuvent être utilisés pour extraire le strontium présent dans une solution aqueuse, notamment une solution acide ou saline provenant d'installations de retraitement de combustibles nucléaires usés.

En effet, les calixarènes de l'invention qui comportent un groupe acétamido dans lequel le groupe amido est un groupement amide tertiaire (R1 et R2 étant des groupes alkyle), sont beaucoup plus efficaces et beaucoup plus sélectifs vis-à-vis du strontium que des calixarènes à groupe amide secondaire (R1 = H, R2 = alkyle) ou amide primaire (R1 = R2 = H).

Par ailleurs, la présence des groupes hydroxy, alcoxy, aryloxy, arylalcoxy ou cycloalcoxy sur les noyaux phényle du calixarène les rend encore plus sélectifs vis-à-vis du strontium. En effet, on a observé que des calixarènes utilisant des groupes alkyle extrayaient le strontium, mais moins fortement que le sodium.

Or, le procédé d'extraction de l'invention est destiné en particulier à extraire le strontium à faible concentration, par exemple 5.10⁻⁴ M, de milieux contenant 4 moles par litre de sodium.

Des calixarènes à substituants alkyle ne peuvent donc être utilisés dans ce cas puisqu'ils extraient davantage le sodium que le strontium.

De plus, les calixarènes de l'invention comportant 6, 7 ou 8 noyaux phényles sont plus efficaces que les calix[4]arènes ou les calix[5]arènes pour l'extraction du strontium en présence de sodium.

Pour réaliser l'extraction du strontium, conformément à l'invention, on met en contact la solution aqueuse contenant le strontium à séparer avec une phase immiscible comprenant au moins un dérivé de calixarène répondant à la formule (I) donnée ci-dessus, pour extraire le strontium dans la phase immiscible.

Cette phase immiscible est généralement constituée par une solution du ou des calixarènes de l'invention dans un solvant organique approprié.

A titre d'exemple de solvants utilisables, on peut citer les alkyl benzènes et les nitrophényl alkyl éthers.

De préférence, on utilise comme solvant un éther tel que l'ortho-nitrophényl hexyl éther.

La concentration en calixarène de la phase liquide immiscible dépend en particulier du solvant utilisé. On peut utiliser des concentrations allant de 10⁻⁴ à 5.10⁻² mol/L, par exemple une concentration de 10⁻² mol/L.

Pour mettre en oeuvre le procédé d'extraction de l'invention, on peut effectuer la mise en contact de la solution aqueuse avec cette phase liquide immiscible dans des installations classiques d'extraction liquide-liquide telles que des mélangeurs-décanteurs, des extracteurs centrifuges, des colonnes pulsées etc.

L'invention peut être mise en oeuvre pour extraire le strontium à partir de solutions aqueuses, notamment de solutions nitriques ayant une concentration en acide nitrique de 1 à 4 mol/L, ainsi que pour extraire le strontium de solutions présentant une forte salinité, par exemple des concentrations en nitrate de sodium de 1 à 4 mol/L.

Il permet, en particulier, d'extraire le strontium à partir de solutions ayant une concentration en strontium très faible par rapport à la concentration en sodium de ces solutions.

D'autres caractéristiques et avantages de l'invention apparaîtront mieux à la lecture des exemples suivants, donnés bien entendu à titre illustratif et non limitatif, en référence aux dessins annexés.

### Brève description des dessins

La figure 1 est un schéma de synthèse des calixarènes de formule (I) correspondant aux exemples 1 à 11.
La figure 2 est un schéma de synthèse des calixarènes de formule (I) correspondant aux exemples comparatifs 12 à 14.
La figure 3 illustre l'évolution du coefficient de distribution D_{Sr} en fonction de la concentration en acide nitrique de la solution de départ (en mol/L), dans le cas du composé 10.
La figure 4 illustre l'évolution des coefficients de distribution D_{Sr} et D_{Na} et de la sélectivité Sr/Na en fonction de la teneur en nitrate de sodium de la solution de départ (en mol/L) et pour une teneur constante en HNO₃ de 1M.
La figure 5 illustre l'évolution des coefficients de distribution D_{Sr} et D_{Na} et de la sélectivité Sr/Na, en fonction de la teneur en nitrate de sodium de la solution aqueuse de départ, en l'absence de HNO3, dans le cas du composé 10.

### Exposé détaillé des modes de réalisation

Les exemples qui suivent illustrent la préparation de dérivés de calixarènes conformes à l'invention.

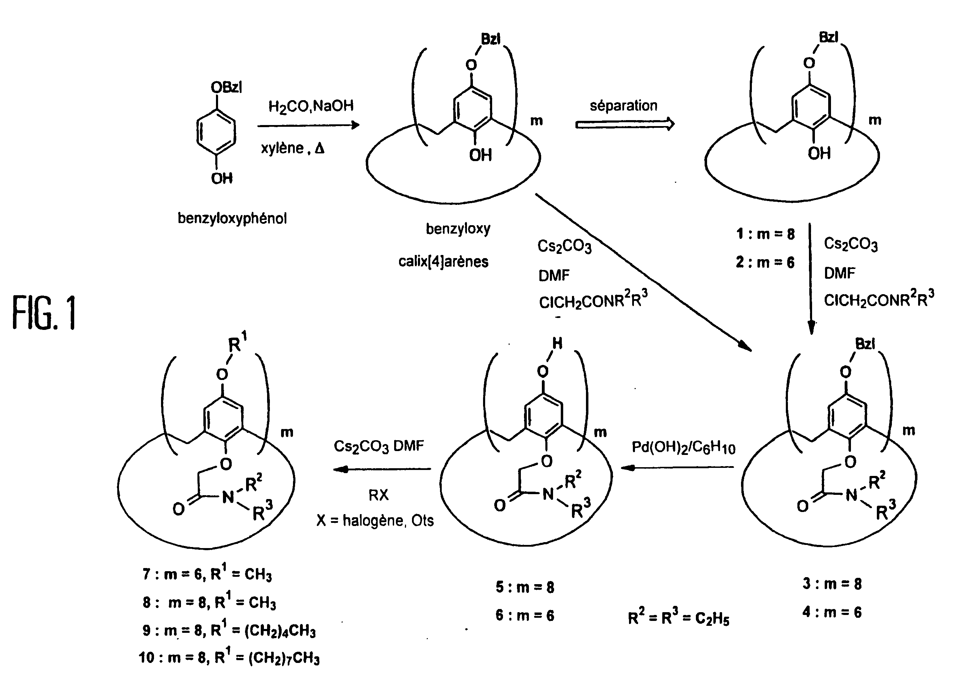

Le schéma de synthèse des exemples 1 à 11 est illustré sur la figure 1 (composés 1 à 10).

### Exemple 1 : préparation d'un mélange de p-benzyloxycalix[n]arènes avec m égal 6, 7 et 8.

On dissout 20,0 g (0,1 mol) de p-benzyloxyphénol dans 400 ml de xylène dans un flacon de 1 litre à fond rond équipé d'un collecteur d'eau de type DEAN & STARK. On chauffe la solution à 100°C, puis on ajoute 1,1 ml (2 mmol) d'une solution aqueuse de NaOH 2N et 5,4 g (179,8 mmol) de paraformaldéhyde. Après 2 heures, on augmente la température à 150°C et un précipité commence de se former. On porte le mélange réactionnel au reflux pendant 48 heures, puis on refroidit et on filtre sur un entonnoir Buchner, ce qui donne un solide correspondant au mélange de p-benzyloxycalix[n]arènes avec m égal 6, 7 et 8.

### Exemple 2 : préparation du 5,11,17,23,29,35,41,47-octabenzyloxy-49,50,51,52,53,54,55,56-octahydroxycalix[8]arène (composé 1).

On lave le produit résultant de la réaction de condensation obtenu dans l'exemple 1 avec de l'éther diéthylique, puis on transfère dans un flacon de 250 ml à fond rond et on met en suspension dans 200 ml de chlorure de méthylène. On porte la solution hétérogène au reflux pendant 3 heures, puis on la filtre à chaud sur un entonnoir Buchner. On obtient ainsi 10,2 g d'un produit blanc constitué par le composé 1, soit un rendement de 48 %.

Les caractéristiques du composé 1 sont les suivantes :
Point de fusion : supérieur à 300°C.
   ¹H NMR (DMSO-D₆) : δ = 8,63 (s, 8H, OH), 7,29 (s, 40H, PhH, 6,60 (s, 16H, ArH), 4,80 (S, 16H, OCH₂Ph), 3,80 (s, 16H, ArCH₂Ar) ;
   ¹³C NMR (DMSO-D₆) : δ = 151,0 (s, Ar ipso), 146,6 (s, Ar para), 137,6 (s, Ph), 128,9 (s, Ar ortho), 128,1, 127,6 (d, Ph), 114,1 (d, Ar meta), 69,7 (t, OCH₂Ph), 32,0 (t, ArCH₂Ar) ;
   MS (CI⁻) 1696 (M⁺) ;
   Analalyse calculée pour C₁₁₂H₉₆O_{16 :} C, 79,23 ; H, 5,69
   Trouvée : C, 79,11 ; H, 5,80.

### Exemple 3 : préparation du 5,11,17,23,29,35-hexabenzyloxy-37,38,39,40,41,42-hexahydroxycalix[6]arène (composé 2).

On part de la liqueur-mère de précipitation du composé 1 obtenu dans l'exemple 2 et on élimine le dichlorométhane par distillation. On traite le résidu au moyen de 50 ml d'éther éthylique. On filtre le précipité formé sur un entonnoir Buchner. On obtient ainsi 1,1 g du composé **2**, ce qui correspond à un rendement de 5 %.

Les caractéristiques du composé **2** sont les suivantes :
Point de fusion : supérieur à 300°C.
¹H NMR (DMSO-D₆, 300K) : δ = 8,52 (s, 6H, OH), 7,29 (m, 30H, PhH), 6,64 (s, 12H, ArH), 4,83 (s, 12H, CH₂Ph), 3,75 (s, 12H, ArCH₂Ar) ;
¹³C NMR DMSO-D₆, 300K) : δ = 151, 7 (s, Ar ipso), 144,9 (s, Ar para), 137,2 (s, Ph), 1 28, 6 (s, Ar ortho), 128,5, 128,1, 127,6 (d, Ph), 114,3 (d, Ar meta), 69,3 (t, OCH₂Ph), 31,2 (t, ArCH₂Ar).
MS (CI+) 1273,4 (M+H⁺) ;
Analyse calculée pour C₈₄H₇₂O₁₂ : C, 79,23 ; H, 5,69
Trouvée : C, 79, 18 ; H, 5,77.

### Exemple 4 : préparation du 5,11,17,23,29,35,41,47-octabenzyloxy-49,50,51,52,53,54,55,56-octakis[(N,N-diéthylamino-carbonyl)méthoxy]calix[8]arène (composé 3).

On part d'une solution comprenant 0,40 mmol du composé **1** dissous dans 20 ml de diméthylformamide DMF sec et on chauffe à 90°C, sous agitation avec Cs₂CO₃ (4 moles pour chaque mole de OH), et 6 moles pour chaque mole de OH de α-chlorodiéthylacétamide.

Après 15 à 20 heures, on introduit dans le mélange réactionnel refroidi 75 ml de HCl 1N et on filtre le produit brut sur un entonnoir de Buchner et on le lave à l'eau.

On dissout le solide dans 20 ml de CH₂Cl₂ et on lave la solution organique avec 2 x 25 ml de HCl 2N et 2 x 25 ml d'eau. On sèche la phase organique sur MgSO₄, on élimine le dichlorométhane par distillation et on purifie le produit obtenu par cristallisation à partir de CH₂Cl₂/CH₃OH à -10°C. On obtient le composé **3** avec un rendement de 61 %.

Les caractéristiques du composé **3** sont les suivantes :
Point de fusion : supérieur à 300°C.
¹H NMR (CDCl₃) δ = 7,13 (m, 40H, PhH), 6,54 (s, 16H, ArH), 4,54 (s, 16H, OCH₂Ph), 4,42 (s, 16H, OCH₂CO), 4,04, (s, 16H, ArHCH₂Ar), 3,25 (q, J = 7Hz, 16 H, NC*H*₂C*H*₃)), 3,12 (q, J = 7 Hz, 16H, NC*H*₂CH₃) 0,99 (t, J = 7 Hz, 24H, NCH₂C*H*₃) 0,93 (t, J = 7Hz, 24H, NCH₂C*H*₃) ;
¹³C NMR CDCl₃ : δ = 166,9 (s, OCH₂CO), 155,0 (s, Ar ipso), 149,2 (s, Ar para), 137,0 (s, Ph), 128,0 (s, Ar ortho), 127,5, 127,4 (d, Ph), 115,0 (d, Ar meta), 72,1 (t, OCH₂CO), 69,4 (t, OCH₂Ph), 41,1, 39,9 (t, N*C*H₂*C*H₃) 30,5 (t, ArCH₂Ar), 14,1, 12,7 (q, NCH₂CH₃) ;
MS (FAB) m/z 2601 (M⁺).
Analyse calculée pour : C₁₆₀H₁₈₄O₂₄N₈ : C, 73,83 ; H, 7,12 ; N, 4,30
Trouvée : C, 73,71 ; H, 7,19 ; N, 4, 48.

### Exemple 5 : préparation du 5,11,17,23,29,35-hexabenzyloxy-37-38,39,40,41,92-hexakis[N,N-diéthylaminocarbonyl)méthoxy]calixa[6]arène (composé 4).

On suit le même mode opératoire que dans l'exemple 4 pour préparer le composé **4** en partant du composé **2**.

On purifie le produit obtenu par cristallisation dans les mêmes conditions et on obtient le composé **4** avec un rendement de 75 %.

Les caractéristiques du composé **4** sont les suivantes :
Point de fusion : 246-247°C.
¹H NMR (CDCl₃, 300K) : δ = 7,52-7,11, 6,81, 6,80, 6,19 et 5,97 (42H, PhH et ArH), 5,07-4,23 (m, 27H, OC*H*₂Ph, OC*H*₂CO, ArC*H*₂Ar), 3,96, 3,79, 3,40 (bs, 25H, NC*H*₂CH₃, ArC*H*₂Ar), 3,08, 2,49(q, 2H, chaque NC*H*₂CH₃), 1,86 (bs, 30 H, NCH₂C*H*₃), 0,75 et -0,51 (t, 3H chaque, NCH₂C*H*₃), 0,90, 0,70, -0, 19, -1, 61 (bs, 1H, NC*H*₂CH₃) ;
¹H NMR (DMSO-d₆, 373K) : δ = 7,31 et 7,24, (s, 30H, PhH), 6,88(bs, 12H, ArH), 4,88 (s, 12H, OC*H*₂Ph, 4,45 (s, 12H, OC*H*₂CO), 3,91 (bs, 12H, ArC*H*₂Ar), 3,26, (bs, 24H, NC*H*₂CH₃, 1,01 (bs, 36H, NCH₂C*H*₃) ;
¹³C NMR (CDCl₃, 300 K) : δ = 168,0, 167,6, 167,5,167,3, 166,9 (s, OCH₂*C*O), 155,1 154,9, 154,3 (s, Ar ipso), 151,4, 150,7, 148,9 (s, Ar para), 138,1, 137,6, 137,1 (s, Ph), 135,4, 134,8, 134,5 (s, Ar ortho), 133,7, 133,5, 128,5, 128,1, 127,9, 127,4, 127,1, 126, 9 (d, Ph), 117, 4, 116, 9, 116,5, 116,0, 115,3, 113,8, 113,5, (d, Ar meta), 74,7, 72,5, 71,4, 70,3, 69,8, 69,2 (t, O*C*H₂CO), 41,62, 41,42, 41,0, 41,0, 40,1, 39,5 (t, N*C*H₂CH₃), 32,5, 32,1, 31,1 (t, ArCH₂Ar), 14,5, 12,9, 12,3, 12,1 (q, NCH₂*C*H₃) ;
Analyse calculée pour C₁₂₀H₁₃₈O₆N₁₈ : C, 73,82 ; H, 7,12 ; N, 4,30
Trouvée : C, 73,78 ; H, 7,18 ; N, 4, 39.
MS (CI) : 1952 (M + 1)⁺

### Exemple 6 : préparation du 5,11,17,23,29,35,41,47-octahydroxy-49,50,51,52,53,54,55,56-octakis[(N,N-diéthylaminocarbonyl)méthoxy]calix[8]arène (composé 5).

On met en suspension 0,5 g du composé **3** obtenu dans l'exemple 4 dans un mélange de 10 ml d'éthanol et de 9 ml de cyclohexène dans un tube Schlenk sous azote. Après addition de 0,1 g, (20 % p/p par rapport au calixarène) de Pd(OH)₂/C (20 %, catalyseur de Pearlman), on chauffe le mélange réactionnel à 90 °C, pendant 15 à 18 heures. On élimine alors le catalyseur par filtration sur un filtre de Célite, on lave soigneusement le filtre avec CH₂Cl₂, C₂H₅OH et de nouveau avec CH₂Cl₂.

On évapore les filtrats collectés sous vide jusqu'à siccité, ce qui laisse un produit brut en rendement quantitatif supérieur à 90 %. Le produit brut peut être cristallisé à partir d'hexane et on obtient ainsi le composé **5** avec un rendement de 90 %.

Les caractéristiques du composé **5** sont les suivantes :
Point de fusion : supérieur à 300°C.
¹H NMR (CDCl₃/CD₃OD = 3/1) : δ = 6,38 (s, 16H, ArH), 4,41 (s, 16H, OCH₂Ph), 3,93 (s, 16H, ArC*H*₂Ar), 3,16, (d, J = 7 Hz, 16H, NC*H*₂CH₃, 3,13 (d, J = 7 Hz, 16H, NC*H*₂CH₃) NC*H*₂CH₃) 1,04 (t, J = 7 Hz, 24H, NCH₂C*H*₃) 0,94 (t, J = 7 Hz, 24H, NCH₂C*H*₃),
¹³C NMR (CDCl₃) : CD₃OD = 3 : 1) : δ = 168,7 (s, OCH₂*C*O), 153,9 (s, Ar ipso), 148,6 (s, Ar para), 135,3 (s, Ar ortho), 116,1 (d, Ar meta), 72,0 (t, OCH₂CO), 42,1, 41,0 (t, N*C*H₂CH₃), 30,9 (t,ArCH₂Ar), 14,2, 12,9 (q, NCH₂*C*H₃),
MS (FAB⁺) m/z 1880,7 (M⁺)
Analyse calculée pour C₁₀₄H₁₃₆O₂₄N₈ : C, 66,37 ; H, 7,27 ; N, 5,95
Trouvée : C, 66,45 ; H, 7,39; N, 6,07.

### Exemple 7 : préparation du 5,11,17,23,29,35-hexahydroxy-37,38,39,40,41,42-hexakis[(N,N-diéthylaminocarbonyl)méthoxy]calix[6]arène (composé 6).

Dans cet exemple, on suit le même mode opératoire que dans l'exemple 6, pour préparer le composé **6** à partir du composé **4**.

On obtient le composé **6** avec un rendement de 80 %.

Les caractéristiques du composé **6** sont les suivantes :
Point de fusion : 255°C (décomposé).
¹H NMR (DMSO-d₆, 300K) : δ = 8,3 (bs, 6H, ArOH), 6,6 (bs, 12H, ArH), 4,21 (bs, 12H, OC*H*₂CO), 3,85, (bs, 12H, ArC*H*₂Ar) 3,30 (bs, 24H, NC*H*₂CH₃), 1,0 (bs, 36H, NCH₂C*H*₃),
¹H NMR (DMSO-d₆, 343K) : δ = 8, 11 (s, 6H, ArOH), 6,35 (s, 12H, ArH), 4,35 (s, 12H, OC*H*₂CO), 3,81, (s, 12H, ArC*H*₂Ar) 3,20 (bs, 24H, NC*H*₂CH₃), 0,90 (bs, 36H, NCH₂C*H*₃),
¹³C NMR (CDCl₃) : δ = 166,8 (s, OCH₂*C*O), 152,5 (s, Ar ipso), 147,8 (s, Ar para), 133,6 (s, Ar ortho), 115,8 (d, Ar meta), 79,4 (t, O*C*H₂CO), 40,71, (t, N*C*H₂CH₃), 30,3 (t, ArCH₂Ar), 13,7, 12,6 (q, NCH₂*C*H₃),
MS (FAB+) m/z 1410,9 (M⁺).
Analyse calculée pour C₇₈H₁₀₂O₆N₁₈ : C, 66,36 ; H, 7,28 ; N, 5,95
Trouvée : C, 66, 44 ; H, 7,33 ; N, 6,02.

### Exemple 8 : préparation du 5,11,17,23,29,35-hexaméthoxy-37,38,39,40,41,42-hexakis[(N,N-diéthylaminocarbonyl)méthoxy]calix[6]arène (composé 7).

A une solution agitée de 0,5 mmol du composé **6** dissous dans 80 ml de DMF sec, on ajoute 3 moles de Cs₂CO₃ pour chaque mole de OH et du iodométhane à raison de 3 mol pour chaque mole de OH. On chauffe le mélange réactionnel à 90°C, sous atmosphère d'azote pendant 18 heures. On élimine alors le DMF sous pression réduite et on refroidit le résidu avec 75 ml d'une solution aqueuse de HCl à 10 %. On ajoute 75 ml de dichlorométhane, on sépare la couche organique et on la lave 2 fois avec 50 ml d'eau. On sèche la phase organique sur MgSO₄ et on élimine le solvant sous pression réduite.

On obtient le composé **7** avec un rendement de 95 %.

Les caractéristiques du composé **7** sont les suivantes :
Point de fusion : 108-110°C
¹H NMR (DMSO-d₆, 300K) : δ = 7,3-6,8 et 5,7 (bs, 12H, ArH), 4,63 (bs, 12H, OC*H*₂CO), 4,3, (bs, 12H, ArC*H*₂Ar), 3,9-3,4 (bs, 42H, OCH₃ et NC*H*₂CH₃), 1,10 (bs, 36H, NCH₂C*H*₃),
¹H NMR (DMSO-d₆, 343K) : δ = 6,70 (s, 12H, ArH), 4,43 (s, 12H, OC*H*₂CO), 3,85, (s, 12H, ArC*H*₂Ar), 3,54 (s, 18H, OCH₃), 3,17 (bs, 24H, NC*H*₂CH₃), 0,96 (bs, 36H, NCH₂C*H*₃) ;
¹³C NMR (CDCl₃, 300K) : δ = 167,1, 166,7, (s, OCH₂CO), 155,9, 155,8 (s, Ar ipso), 148,5, 148,3 (s, Ar para), 136,2, 135,3, 135,1, 134,5, 134,2 (s, Ar ortho), 116,6, 116,0, 115,7, 114,8, 114,6, 113,9 (d, Ar meta), 72, 4, 72,2 (t, O*C*H₂CO), 57,4, 56,8, 55,3 (q, OCH₃), 41,2, 39,9 (t, N*C*H₂*C*H₃), 31,7 (t, ArCH₂Ar), 14,5, 12,8 (q, N*C*H₂*C*H₃),
Analyse calculée pour C₈₄H₁₁₄N₆O₁₈ : C, 67,44 ; H, 7,68 ; N, 5,62
Trouvée : C, 67, 38 ; H, 7,75; N, 5,73.
MS (CI+) = 1496 (M+H⁺).

### Exemple 9 : préparation du 5,11,17,23,29,35,41,47-octaméthoxy-49,50,51,52,53,54,55,56-octakis[(N,N-diéthylaminocarbonyl)méthoxy]calix[8]arène (composé 8).

On suit le même mode opératoire que dans l'exemple 8 pour préparer le composé **8** à partir du composé **5** en utilisant également l'iodométhane CH₃I.

On obtient ainsi le composé **8** avec un rendement de 70 %.

Les caractéristiques de ce composé sont les suivantes :
Point de fusion : 96-97°C .
¹H NMR CDCl₃) : δ = 6,43 (s, 16H, ArH), 4,43 (s, 16H, OCH₂CO), 4,04, (s, 16H, ArCH₂Ar) 3,45 (s, 24H, OCH₃), 3,29 (d, 16H, NC*H*₂CH₃), J = 6,3 Hz), 3,13 (d, 16H, NC*H*₂CH₃, J = 6,3 Hz), 1,04 (t, 24H, NCH₂C*H*₃), J = 6 Hz), 0,90 (t, 24H, NCH₂C*H*₃, J = 6 Hz) ;
¹³C NMR CDCl₃ : δ = 166,9 (s, OCH₂*C*O), 155,9 (s, Ar ipso), 148,9 (s, Ar para), 134,6 (s, Ar ortho), 113, 9, (d, Ar meta), 72,0 (t, O*C*H₂CO), 54,9 (s, OCH₃), 41, 0, 39, 8 (t, N*C*H₂CH₃), 30,5 (t, ArCH₂Ar), 14, 0, 12, 6 (q, NCH₂*C*H₃) ;
Analyse calculée pour C₁₁₂H₁₅₂N₈O₂₄ : C, 67,45 ; H, 7,68 ; N, 5,62
Trouvée : C, 67,54 ; H, 7,56 ; N, 5, 77
MS (CI+) : m/z = 1994 (M+H⁺).

### Exemple 10 : préparation du 5,11,17,23,29,35,41,47-octapentoxy-49,50,51,52,53,54,55,56-octakis[(N,N-diéthylaminocarbonyl)méthoxy]calix[8]arène (composé 9).

On suit le même mode opératoire que dans l'exemple 8 pour préparer le composé **9** à partir du composé **5**, en utilisant comme agent alkylant le n-CH₃(CH₂)₄I.

On obtient le composé **9** pur par chromatographie flash (SiO₂ : AcOEt/MeOH = 10/1) avec un rendement de 65 %.

Les caractéristiques de ce composé sont les suivantes :
Point de fusion : 68-70°C.
¹H NMR CDCl₃) : δ = 6,49 (s, 16H, ArH), 4,37 (s, 16H, OCH₂CO), 4,00 (s, 16H, ArCH₂Ar), 3,65 (bs, 16H, OCH₂R), 3,24 ((bs, 16H, NC*H*₂CH₃), 3,11 (bs, 16H, NC*H*₂CH₃), 1,55 (bs, 48H, (CH₂)_{3,}), 1,28 (bs, 24H, RCH₃), 1,01 (t, 24H, NCH₂C*H*₃), 0,85 (t, 24H, NCH₂C*H*₃),
¹³C NMR (CDCl₃) : δ = 166,9 (s, OCH₂*C*O), 155,3 (s, Ar ipso), 148,9 (s, Ar para), 134,3, (s, Ar ortho), 114,6 (d, Ar meta), 72,1 (t, O*C*H₂CO), 67,7 (s, OCH₂R), 41,0, 39,8 (t, N*C*H₂CH₃), 30,4, (t, ArCH₂Ar), 28, 8 (t, *C*H₂CH₂CH₂CH₃), 28,7 (t, *C*H₂CH₂CH₃), 22, 3 (t, *C*H₂CH₃), 13,8 (q, CH₃), 14,0, 12,6 (q, N*C*H₂CH₃) ;
Analyse calculée pour C₁₄₄H₂₁₆N₈O₂₄ : C, 70,79 ; H, 8,91 ; N, 4,59
Trouvée : C, 70,83 ; H, 8,86 ; N, 4,66
MS (CI+) : m/z = 2443 (M + 1)⁺.

### Exemple 11 : préparation du 5,11,17,23,29,35,41,47-octaoctoxy-49,50,51,52,53,54,55,56-octakis[(N,N-diéthylaminocarbonyl)méthoxy]calix[8]arène (composé 10).

Dans cet exemple, on suit le même mode opératoire que dans l'exemple 8 en partant du composé **5** et en utilisant comme agent d'alkylation le n-CH₃(CH₂)₇ Ots.

On obtient le composé **10** à l'état pur par chromatographie flash (SiO₂ : AcOEt/MeOH = 10/0,1), avec un rendement de 57 %.

Les caractéristiques du composé **10** sont les suivantes :
Point de fusion : 68-70°C .
¹H NMR (CDCl₃) : δ = 6,47 (bs, 16H, ArH), 4,34 (bs, 16H, OCH₂CO), 3,99 (s, 16H, ArCH₂Ar) 3,65 (bs, 16H, OCH₂R), 3,22 ((bs, 32H, NC*H*₂CH₃), 1,23 (bs, 96H, (C*H*₂)₆CH₃), 0,90 (bs, 24H, NC*H*₂CH₃), 0,86 (bs, 24H, NCH₂C*H*₃), 0,83 (t, J = 6,5 Hz, 24H, (CH₂)₇C*H*₃),
¹³C NMR (CDCl₃) : δ = 166,9 (s, OCH₂CO), 155,2 (s, Ar ipso), 148,9 (s, Ar para), 134,4 (s, Ar ortho), 114,6, (d, Ar meta), 72,0 (t, O*C*H₂CO), 67,7 (s, OCH₂R), 41,0, 39,8 (t, N*C*H₂CH₃), 31,7 (t, *C*H₂CH₂CH₂CH₂ CH₂CH₃), 30,4 (t, ArCH₂Ar), 29,5 (t, *C*H₂CH₂CH₂CH₂CH₃), 29,1 (t, *C*H₂CH₂CH₂CH₃), 25,9 (t, *C*H₂CH₂CH₃), 22,4 (t, *C*H₂CH₃), 13,8 (q, CH₃) ; 14,0, 12,6 (q, NCH₂*C*H₃),
Analyse calculée pour C₁₆₈H₂₆₄N₈O₂₄ : C, 72,58 ; H, 9,57 ; N, 4,03
Trouvée : C, 72,62 ; H, 9,51 ; N, 4,12.
MS (CI+) : m/z = 27779(M + 1)⁺

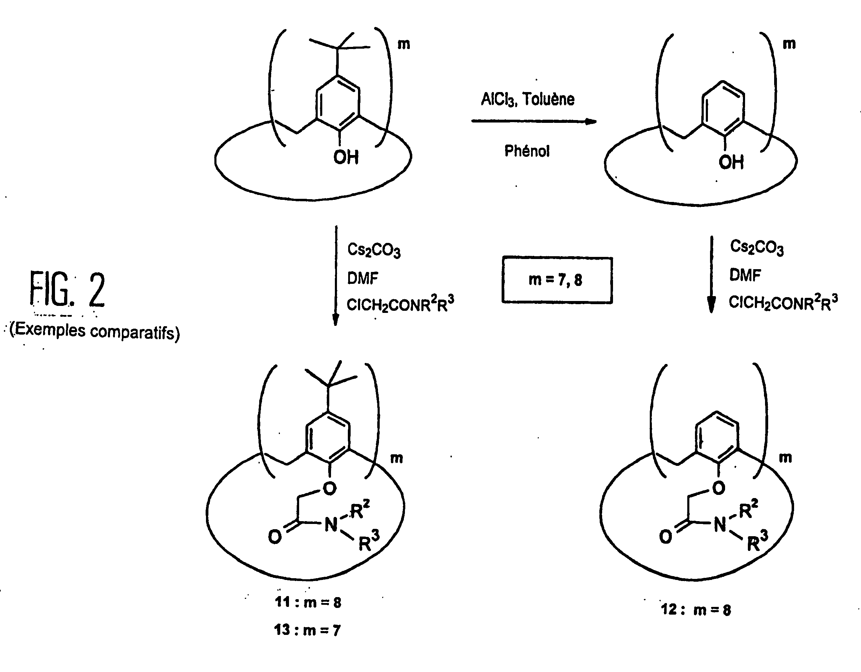

Le schéma de synthèse des exemples 12 à 14 est illustré sur la figure 2.

### Exemple comparatif 12 : préparation du 5,11,17,23,29,35,41,47-octakis(1,1-diméthyléthyl)-49,50,51,52,53,54,55,56-octakis[(N,N-diéthylaminocarbonyl)méthoxy]calix[8]arène (composé 11)

Dans cet exemple, on suit le même mode opératoire que dans l'exemple 4 pour préparer le composé **11** à partir de 5,11,17,23,29,35,41,47-octakis(1,1-diméthyléthyl)calix[8]arène, soit le composé de formule (III) avec R¹ représentant le groupe tert-butyle et m égal à 8.

On obtient ainsi le composé **11** à l'état pur par cristallisation à partir de méthanol, avec un rendement de 64 %.

Les caractéristiques du composé **11** sont les suivantes :
Point de fusion : supérieur à 300°C.
¹H NMR (CDCl₃) : δ = 6,69 (s, 16H, ArH), 4,37 (s, 16H, OCH₂CO), 4,13, (s, 16H, ArCH₂Ar) 3,21 (bs, 16H, NC*H*₂CH₃), 1,05 (s, 72H, tBu), 1,22 (t, 24H, J = 7 Hz, NC*H*₂CH₃), 0,84 (t, 24H, J = 7 Hz, NCH₂C*H*₃),
¹³C NMR (CDCl₃) : δ = 167,0 (s, OCH₂*C*O), 153,4 (s, Ar ipso), 146,0 (s, Ar para), 132,5, (s, Ar ortho), 125,9 (d, Ar meta), 71,1 (t, O*C*H₂CO), 41,1, 39,9 (t, N*C*H₂CH₃), 34,1 (s, C(CH₃)₃), 31,2 (t, ArCH₂Ar), 31,4 (s, C(CH₃)₃), 14,0, 12,8 (q, NCH₂*C*H₃) ;
Analyse calculée pour C₁₃₆H₂₀₀N₈O₁₆ : C, 74,14 ; H, 9,15; N, 5,09
Trouvée : C, 74,03 ; H, 9,22 ; N, 5,18
MS (CI+) = 2201 (M⁺).

### Exemple comparatif 13 : préparation du 49,50,51,52,53,54,55,56-octakis[(N,N-diéthylaminocarbonyl)méthoxy]calix[8]arène (composé 12).

On suit le même mode opératoire que dans l'exemple 4 pour préparer le composé **12** à partir de calix[8]arène. On obtient le composé **12** pur par chromatographie flash (SiO₂, AcOEt/MEt₃ = 10/0,5) et cristallisation à partir d'acétate d'éthyle froid, avec un rendement de 75 %.

Les caractéristiques du composé **12** sont les suivantes :
Point de fusion : 84-86°C.
¹H NMR CDCl₃) : δ = 6,61 (bs, 24H, ArH), 4,40 (s, 16H, OCH₂CO), 4,10 (s, 16H, ArCH₂Ar), 3,31-3,01 (m, 32H, NC*H*₂CH₃), 1,10 - 0,77 (m, 48H, NC*H*₂CH₃),
¹³C NMR (CDCl₃) : δ = 166,8 (s, OCH₂*C*O), 155,4 (s, Ar ipso), 133,8 (s, Ar ortho), 129,0, (s, Ar meta), 124,3 (d, Ar para), 71,5 (t, O*C*H₂CO), 40,9, 39,7 (t, N*C*H₂CH₃), 30,4 (t, ArCH₂Ar), 13,9, 12,7 (q, N*C*H₂CH₃) ;
Analyse calculée pour C₁₀₄H₁₃₆N₈O₁₆ : C, 71,20 ; H, 7,81 ; N, 6,39
Trouvée : C, 71,15 ; H, 7,79 ; N, 6,44
MS (CI) = 1753 (M)⁺.

### Exemple comparatif 14 : préparation du 5,11,17,23,29,35,41-heptakis(1,1-diméthyléthyl)-43,44,45,46,47,48,49-heptakis [(N,N-diéthylaminocarbonyl)méthoxy]calix[7]a-rène (composé 13).

On obtient ce composé **13** en suivant le mode opératoire de l'exemple 4 à partir à partir de 5,11,17,23,29,35,41-heptakis(1,1-diméthyléthyl)calix[7]arène.

Ce calixarène a été préparé selon le procédé décrit par Shinkai, Bull. Chem. Soc. Jpn, 1994, 10499 [3] ; Markoxitz JACS, 1989, 111, 81-92 [4].

On obtient le composé **13** à l'état pur par cristallisation à partir de méthanol, avec un rendement de 70 %.

Les caractéristiques de ce composé sont les suivantes :
Point de fusion : 248-250°C.
¹H NMR (CDCl₃) : δ = 7,03 (s, 14H, ArH), 4,48 (s, 14H, OCH₂CO), 4,04, (s, 14H, ArCH₂Ar), 3,38-3,23 (m, 28H, NC*H*₂CH₃), 1,03 (s, 63H, tBu), 1,00 (t, 42H, J = 7 Hz, NCH₂C*H*₃) ;
¹³C NMR (CDCl₃) : δ = 167,2 (s, OCH₂CO), 153,4 (s, Ar ipso), 147,1 (s, Ar para), 132,6 (s, Ar ortho), 126,4, (d, Ar meta), 72,1 (t, O*C*H₂CO), 40,1, 39,9 (t, N*C*H₂CH₃), 34,0 (s, C(CH₃)₃), 31,4 (s, C(CH₃)₃), 31,2 (t, ArCH₂Ar), 14,5, 12,9 (q, NCH₂*C*H₃) ;
Analyse calculée pour C₁₁₉H₁₇₅N₇O₁₄ : C, 74,14 ; H, 9,15 ; N, 5,09
Trouvée : C, 74,07 ; H, 9, 20 ; N, 5, 13
MS (CI+) :1926,8 (M⁺).

### Exemple 15

Dans cet exemple, on teste l'efficacité des composés de l'invention pour extraire les métaux M suivants : césium-137, strontium-85 et sodium-22, à partir d'une solution aqueuse nitrique ayant une concentration en acide nitrique de 1M contenant 5.10⁻⁴ M des métaux M à extraire.

Dans cet exemple, on met en contact 3 ml de chaque solution aqueuse contenant un seul des métaux à extraire avec 3 ml d'une solution organique constituée par du nitrophényl hexyléther (NPHE) contenant 10⁻² mol/L du calixarène testé. On effectue la mise en contact dans un tube en polypropylène de 20 ml que l'on soumet à une agitation.

Après 1 heure de mise en contact, on laisse décanter les deux phases et on mesure leurs teneurs respectives en sodium, strontium et césium

On détermine alors le coefficient de distribution D_{M} qui correspond au rapport de la concentration de l'élément M dans la phase organique sur la concentration de ce même élément M dans la phase aqueuse.

Les résultats obtenus et les calixarènes testés sont donnés dans le tableau 1.

### Exemple 16

Dans cet exemple, on teste les propriétés d'extraction des calixarènes de l'invention vis-à-vis du cobalt-60, du strontium-85, du césium-137 et de l'europium-152 présents dans une solution aqueuse contenant 10⁻² mol/L de HNO₃ et 4 mol/L de NaNO₃. On suit le même mode opératoire que dans l'exemple 15 pour assurer l'extraction avec les composés répertoriés dans le tableau 2 qui sont à une concentration de 10⁻² mol/L dans NPHE.

Les résultats obtenus sont donnés dans le tableau 2.

### Exemple 17

Dans cet exemple, on évalue l'efficacité des calixarènes de l'invention pour extraire ⁶⁰Co, ⁸⁵Sr, ¹³⁷Cs et ¹⁵²Eu, à partir d'une solution aqueuse ayant une concentration en acide nitrique de 1 mol/L et contenant 4 mol/L de NaNO₃.

Le mode opératoire est identique à celui de l'exemple 15 et les résultats obtenus ainsi que les calixarènes utilisés sont mentionnés dans le tableau 3.

### Exemple 18

Dans cet exemple, on teste l'efficacité du calixarène (composé 10) pour l'extraction du strontium à partir de solutions aqueuses nitriques ayant des concentrations variables (10⁻³, 10⁻², 10⁻¹, 1 et 10 M) en acide nitrique.

On suit le même mode opératoire que dans l'exemple 15 avec le composé 10 à une concentration de 10⁻² mol/L dans NPHE.

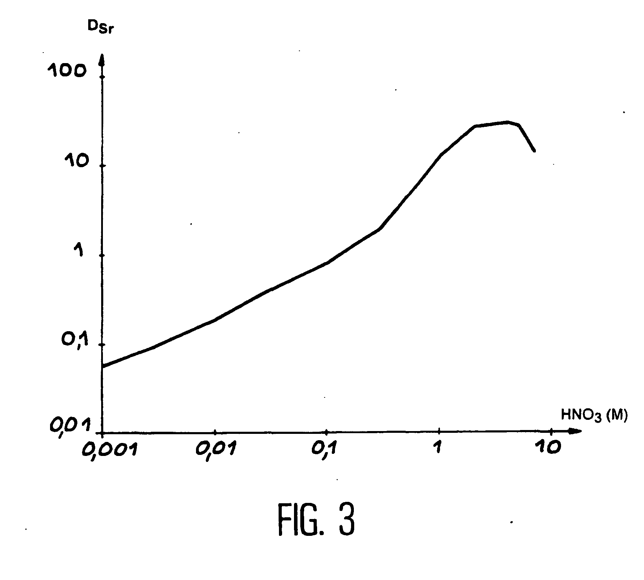

Les résultats obtenus sont donnés sur la figure 3 qui illustre l'évolution du coefficient de distribution D_{Sr} en fonction de la concentration en acide nitrique.

### Exemple 19

Dans cet exemple, on teste l'efficacité du calixarène (composé 10) pour l'extraction du strontium et du sodium à partir de solutions aqueuses ayant une concentration en acide nitrique de 1 M et des concentrations variables (0,5, 1, 2, 3 et 4 M) en NO₃Na. On suit le même mode opératoire que dans l'exemple 15 en utilisant une phase organique constituée par le composé 10 à une concentratyion de 10⁻² mol/L dans NPHE.

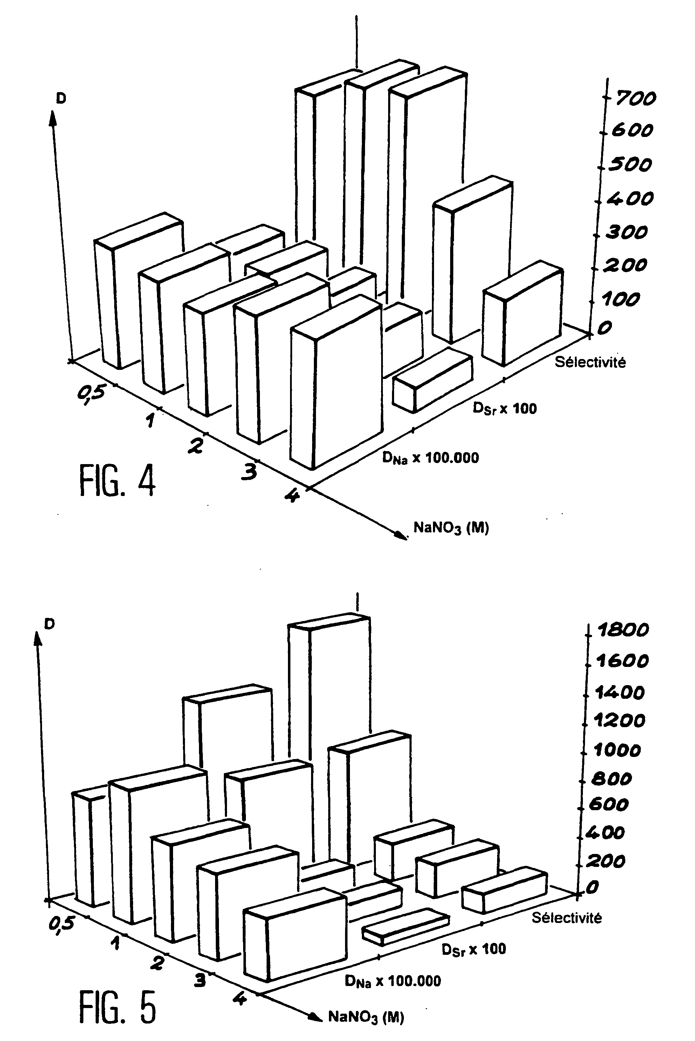

Les résultats obtenus sont donnés sur la figure 4 qui illustre l'évolution de D_{Sr}, D_{Na} et de la sélectivité Sr/Na, en fonction de la concentration en NaNO₃.

### Exemple 20:

Dans cet exemple, on teste l'efficacité du calixarène (composé 10) pour l'extraction du strontium et du sodium à partir de solutions aqueuses sans acide nitrique ayant des concentrations variables (0,5, 1, 2, 3 et 4 M) en NO₃Na. On suit le même mode opératoire que dans l'exemple 15 en utilisant une phase organique constituée par le composé 10 à une concentration de 10⁻² mol/L dans NPHE.

Les résultats obtenus sont donnés sur la figure 5 qui illustre l'évolution de D_{Sr}, D_{Na} et de la sélectivité Sr/Na, en fonction de la concentration en NaNO₃.

### REFERENCES CITEES

[1] : WO94/12502.
[2] : WO94/24138.
[3] : Shinkai, Bull. Chem. Soc. Jpn, 1994, 10499.
[4] : Markoxitz JACS, 1989, 111, 81-92.

**TABLEAU 1**

| **Exemple 15** | | | | |
|---|---|---|---|---|
| Calixarène dans NPHE 10⁻² M | M(NO₃)ₙ5.10⁻⁴ M-HNO₃1M | | | |
| | ²²Na | ⁸⁵Sr | ¹³⁷Cs | Sr/Na |
| Composé 3 | < 10⁻³ | 20 | < 10⁻³ | > 20000 |
| Composé 8 | < 10⁻³ | 6,5 | < 10⁻³ | 6500 |
| Composé 9 | < 10⁻³ | 30 | < 10⁻³ | >30000 |
| Composé 10 | < 10⁻³ | 24 | < 10⁻³ | > 24000 |
| Composé 11 (exemple comparatif) | Précipitation | | | |
| Composé 12 (exemple comparatif) | 6.10⁻³ | 8,3 | 10⁻² | 1400 |
| Composé 4 | < 10⁻³ | 2,9 | < 10⁻³ | > 2900 |
| Composé 13 (exemple comparatif) | 0,04 | 1,13 | | 28 |

**TABLEAU 2**

| **Exemple 16** | | | | |
|---|---|---|---|---|
| Calixarène dans NPHE 10⁻² M | NaNO₃ 4M - HNO₃10⁻² M | | | |
| | ⁶⁰Co | ⁸⁵Sr | ¹³⁷Cs | ¹⁵²Eu |
| Composé 3 | < 10⁻³ | 1,5 | 6.10⁻² | < 10⁻³ |
| Composé 8 | < 10⁻³ | 0,8 | < 10⁻³ | < 10⁻³ |
| Composé 9 | < 10⁻³ | 1,5 | < 10⁻³ | 4.10⁻² |
| Composé 10 | < 10⁻³ | 1,3 | 9.10⁻³ | 9.10⁻² |
| Composé 11 (exemple comparatif) | < 10⁻³ | 8.10⁻² | 1,6.10⁻² | < 10⁻³ |
| Composé 12 (exemple comparatif) | < 10⁻³ | 0,5 | 3.10⁻³ | < 10⁻³ |

**TABLEAU 3**

| **Exemple 17** | | | | |
|---|---|---|---|---|
| Calixarène dans NPHE 10⁻² M | NaNO₃ 4M - HNO₃1M | | | |
| | ⁶⁰Co | ⁸⁵Sr | ¹³⁷Cs | ¹⁵²Eu |
| Composé 3 | < 10⁻³ | 5 | 0,1 | < 10⁻³ |
| Composé 8 | < 10⁻³ | 2,2 | 0,28 | < 10⁻³ |
| Composé 9 | < 10⁻³ | 0,34 | 0,14 | < 10⁻³ |
| Composé 10 | < 10⁻³ | 3,8 | 0,27 | < 10⁻³ |
| Composé 11 (exemple comparatif) | < 10⁻³ | 0,4 | 0,2 | < 10⁻³ |
| Composé 12 (exemple comparatif) | < 10⁻³ | 1,3 | 3.10⁻² | < 10⁻³ |
| Composé 4 | < 10⁻³ | 1,8 | < 10⁻³ | < 10⁻³ |
| Composé 13 (exemple comparatif) | | 0,13 | | |

## Revendications

1. Dérivé de calixarène de formule : 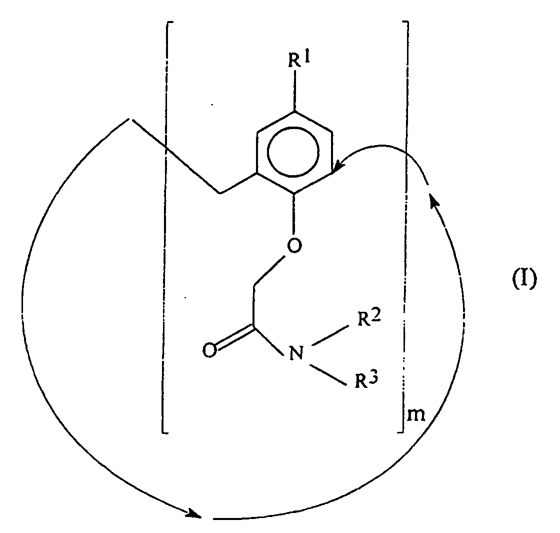 dans laquelle :
- R¹ représente un groupe hydroxyle, alcoxy, aryloxy, arylalcoxy, ou cycloalcoxy, ou un groupe de formule O(CH₂)ₙ[O(CH₂)ₚ]_{q} OR⁴ (II) dans laquelle R⁴ représente un atome d'hydrogène ou un groupe alkyle, n et p sont des nombres entiers allant de 1 à 6, et q est égal à 0 ou est un nombre entier de 1 à 6,
- R² et R³ qui peuvent être identiques ou différents, représentent un groupe alkyle, cycloalkyle ou aryle, ou un groupe de formule : O(CH₂)ₙ [O(CH₂)ₚ]_{q} OR⁴ (II) dans laquelle R⁴, n, p et q sont tels que définis ci-dessus, ou
- R² et R³ forment avec l'atome d'azote auquel ils sont liés un groupe hétérocyclique choisi parmi les groupes pipéridyle, pyrrolidinyle, morpholinyle, thiomorpholinyle, tétrahydropyridyle, imidazolidinyle, indolinyle, tétrahydroquinolyle et perhydroindolyle, et
- m est un nombre entier allant de 6 à 8.

2. Dérivé de calixarène selon la revendication 1, dans lequel R¹ est un groupe alcoxy ou arylalcoxy.

3. Dérivé de calixarène selon la revendication 2, dans lequel le groupe alcoxy est le groupe pentoxy ou octoxy.

4. Dérivé de calixarène selon la revendication 2, dans lequel le groupe arylalcoxy est le groupe benzyloxy.

5. Dérivé de calixarène selon l'une quelconque des revendication 1 à 4, dans lequel R² et R³ sont des groupes alkyle.

6. Dérivé de calixarène selon l'une quelconque des revendications 1 à 5, dans lequel m est égal à 6 ou 8.

7. Dérivé de calixarène choisi parmi les :
5,11,17,23,29,35,41,47-octabenzyloxy-49,50,51,52,53,54,55,56-octakis [(N,N-diéthylaminocarbonyl)méthoxy]calix[8]arène,
5,11,17,23,29,35,41,47-octaméthoxy-49,50,51,52,53,59,55,56-octakis [(N,N-diéthylaminocarbonyl)méthoxy]calix[8]arène,
5,11,17,23,29,35-hexaméthoxy-37,38,39,40,41,42-hexakis [(N,N-diéthylamino-carbonyl)méthoxy]-calix[6]arène,
5,11,17,23,29,35-hexabenzyloxy-37,38,39,40,41,42-hexakis [(N,N-diéthylaminocarbonyl)méthoxy]calix[6]arène,
5,11,17,23,29,35,41,47-octakis(1,1-diméthyl éthyl)-49,50,51,52,53,54,55,56-octakis [(N,N-diéthylaminocarbonyl)méthoxy]calix[8]arène,
49,50,51,52,53,54,55,56,-octakis [(N,N-diéthylaminocarbonyl)méthoxy]calix[8]arène
5,11,17,23,29,35,41,47-octapentoxy-49,50,51,52,53,54,55,56-octakis [(N,N-diéthylaminocarbonyl)méthoxy]calix[8]arène,
5,11,17,23,29,35,41,47-octaoctoxy-49,50,51,52,53,54,55,56-octakis [(N,N-diéthylaminocarbonyl)méthoxy]calix[8]arène,
5,11,17,23,29,35,41,47-octahydroxy-49,50,51,52,53,54,55,56-octakis [(N,N-diéthylaminocarbonyl)méthoxy]calix[8]arène,
- 5,11,17,23,29,35,41-heptakis(1,1-diméthyléthyl)-43,44,45,46,47,48,49-heptakis [(N,N-diéthylaminocarbonyl)méthoxy]calix[8]arène, et
- 5,11,17,23,29,35-hexahydroxy-37,38,39,40,41,42-hexakis [(N,N-diéthylamino-carbonyl)méthoxy]calix[6]arène.

8. Procédé de préparation d'un dérivé de calixarène de formule : 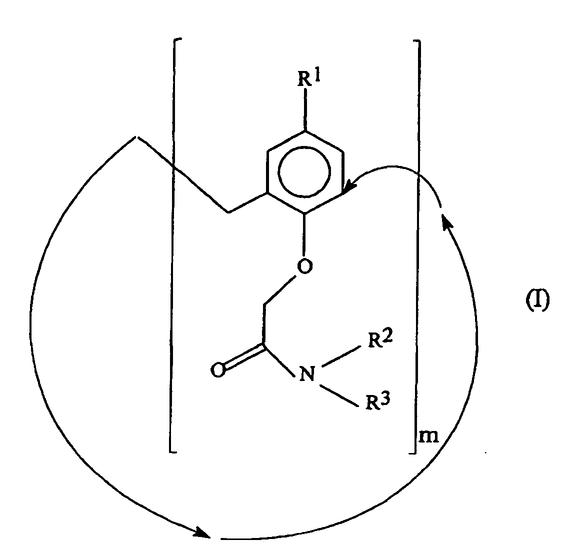 dans laquelle :
- R¹ représente un atome d'hydrogène ou un groupe benzyloxy,
- R² et R³ qui peuvent être identiques ou différents, représentent un groupe alkyle, cycloalkyle ou aryle, ou un groupe de formule : O(CH₂)ₙ [O(CH₂)ₚ]_{q} OR⁴ (II) dans laquelle R⁴, n, p et q sont tels que définis ci-dessus, ou
- R² et R³ forment avec l'atome d'azote auquel ils sont liés un groupe hétérocyclique choisi parmi les groupes pipéridyle, pyrrolidinyle, morpholinyle, thiomorpholinyle, tétrahydropyridyle, imidazolidinyle, indolinyle, tétrahydroquinolyle et perhydroindolyle, et
- m est un nombre entier allant de 6 à 8.
qui comprend la réaction d'un calixarène de formule : 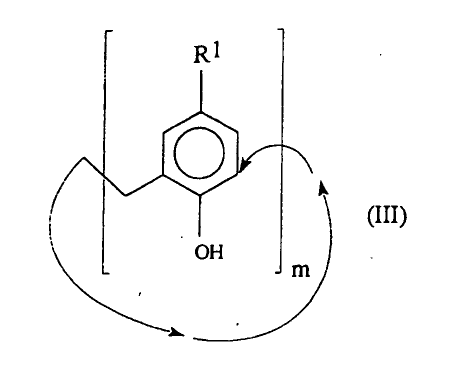 dans laquelle R¹ et m sont tels que définis ci-dessus avec un chloroacétamide de formule : 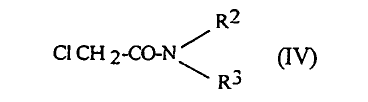 dans laquelle R² et R³ sont tels que définis ci-dessus.

9. Procédé de préparation d'un dérivé de calixarène de formule : 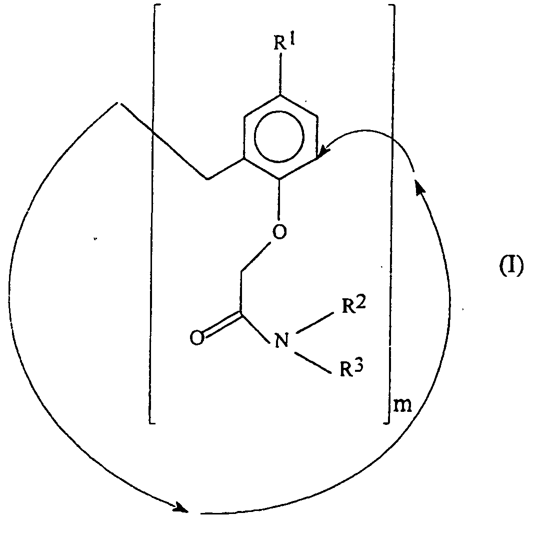 dans laquelle :
- R¹ représente un groupe alcoxy,
- R² et R³ qui peuvent être identiques ou différents, représentent un groupe alkyle, cycloalkyle ou aryle, ou un groupe de formule : O(CH₂)ₙ [O(CH₂)ₚ]_{q} OR⁴ (II) dans laquelle R⁴, n, p et q sont tels que définis ci-dessus, ou
- R² et R³ forment avec l'atome d'azote auquel ils sont liés un groupe hétérocyclique choisi parmi les groupes pipéridyle, pyrrolidinyle, morpholinyle, thiomorpholinyle, tétrahydropyridyle, imidazolidinyle, indolinyle, tétrahydroquinolyle et perhydroindolyle, et
- m est un nombre entier allant de 6 à 8.
qui comprend les étapes suivantes :
a) la réaction d'un calixarène de formule : 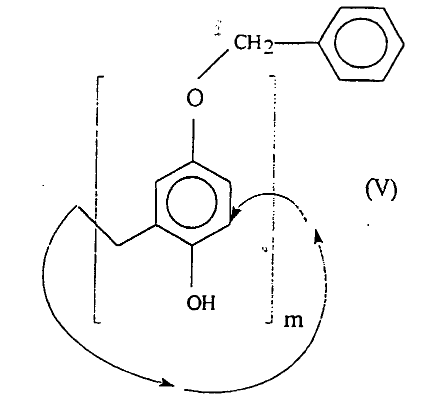 dans laquelle m est tel que défini ci-dessus, avec un chloroacétamide de formule : 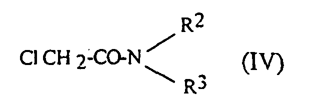 dans laquelle R² et R³ sont tels que définis ci-dessus, pour obtenir le dérivé de formule : 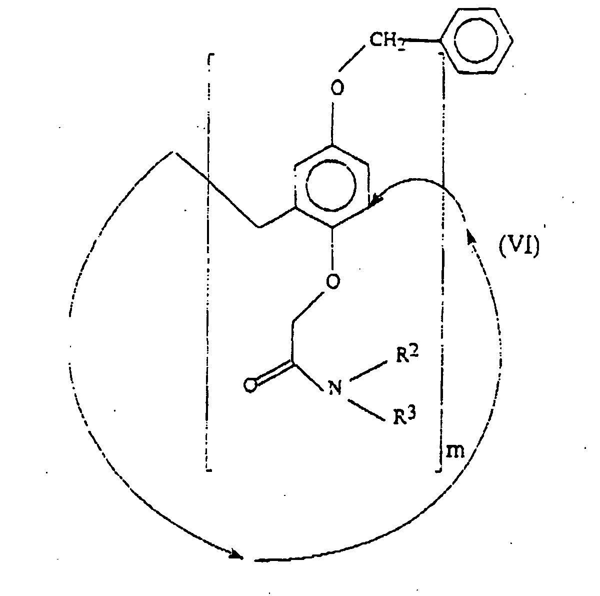 dans laquelle R² et R³ sont tels que définis ci-dessus,
b) réaction du dérivé de formule (VI) avec Pd(OH₂) pour obtenir le calixarène de formule : 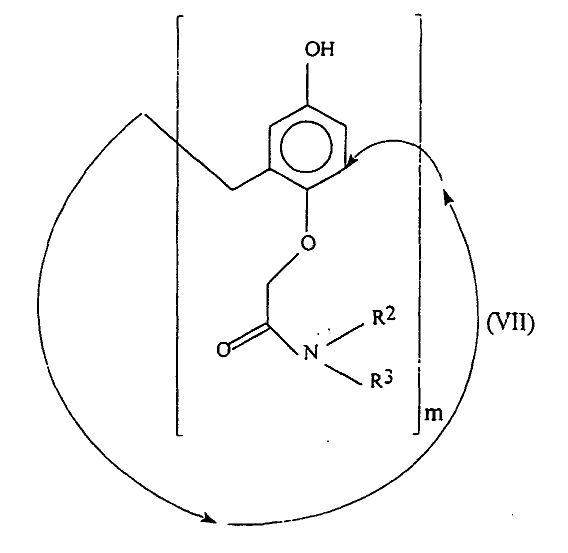
c) réaction du calixarène de formule VII avec un dérivé halogéné de formule R⁵X dans laquelle R⁵ représente un groupe alkyle et X est un atome d'halogène pour obtenir le dérivé de calixarène de formule (I) dans laquelle R¹ représente le groupe alcoxy OR⁵.

10. Procédé de préparation d'un dérivé de calixarène de formule : 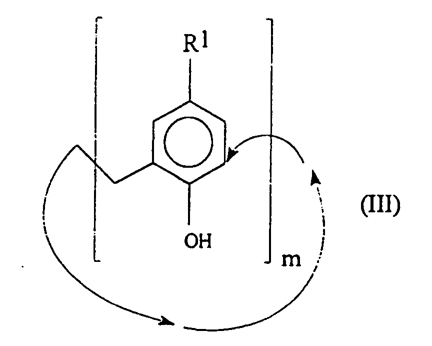 dans laquelle R¹ représente le groupe benzyloxy et m est égal à 6 ou 8, par réaction de condensation du p-benzyloxyphénol de formule : 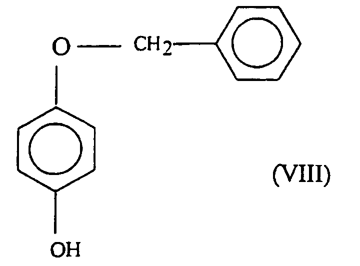 pour obtenir un mélange des calix[6]arène, calix[7]arène et calix[8]arène de formule (III), et séparation du calixarène de formule (III) avec m = 6 ou 8.

11. Procédé pour extraire le strontium présent dans une solution aqueuse, qui consiste à mettre en contact la solution aqueuse avec une phase immiscible comprenant au moins un dérivé de calixarène de formule : 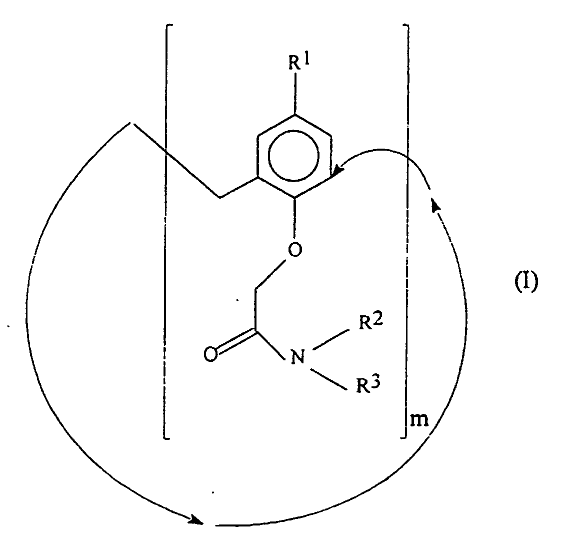 dans laquelle :
- R¹ représente un atome d'hydrogène, un groupe alkyle, alcoxy, aryle, aryloxy, arylalkyle, arylalcoxy, cycloalkyle ou cycloalcoxy, ou un groupe de formule O(CH₂)ₙ[O(CH₂)ₚ]_{q} OR⁴ (II) dans laquelle R⁴ représente un atome d'hydrogène ou un groupe alkyle, n et p sont des nombres entiers allant de 1 à 6, et q est égal à 0 ou est un nombre entier de 1 à 6,
- R² et R³ qui peuvent être identiques ou différents, représentent un groupe alkyle, cycloalkyle ou aryle, ou un groupe de formule : O(CH₂)ₙ [O(CH₂)ₚ]_{q} OR⁴ (II) dans laquelle R⁴, n, p et q sont tels que définis ci-dessus, ou
- R² et R³ forment avec l'atome d'azote auquel ils sont liés un groupe hétérocyclique choisi parmi les groupes pipéridyle, pyrrolidinyle, morpholinyle, thiomorpholinyle, tétrahydropyridyle, imidazolidinyle, indolinyle, tétrahydroquinolyle et perhydroindolyle, et
- m est un nombre entier allant de 6 à 8,
pour extraire le strontium dans la phase immiscible.

12. Procédé selon la revendication 11, dans lequel la solution aqueuse est une solution nitrique comprenant de 1 à 4 mol/l d'acide nitrique ou une solution saline ayant une concentration en nitrate de sodium de 1 à 4 mol/L.

13. Procédé selon la revendication 11 ou 12, dans lequel la phase immiscible est une solution du dérivé de calixarène de formule (I) dans un solvant organique.

14. Procédé selon la revendication 13, dans lequel le solvant organique est un nitrophénylalkyléther.

## Patentansprüche

1. Calixaren-Derivat der Formel: 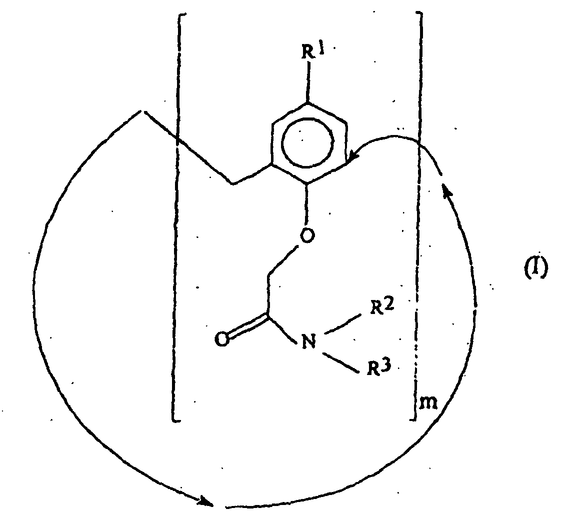 worin bedeuten:
- R¹ eine Hydroxyl-, Alkoxy-, Aryloxy-, Arylalkoxy- oder Cycloalkoxy-Gruppe oder eine Gruppe der Formel O(CH₂)ₙ[O(CH₂)ₚ]_{q}OR⁴ (II), in der R⁴ für ein Wasserstoffatom oder eine Alkylgruppe steht, n und p ganze Zahlen von 1 bis 6 darstellen und q für die Zahl 0 oder eine ganze Zahl von 1 bis 6 steht,
- R² und R³, die gleich oder verschieden sein können, eine Alkyl-, Cycloalkyl- oder Arylgruppe oder eine Gruppe der Formel O(CH₂)ₙ[O(CH₂)ₚ]_{q}OR⁴ (II), in der R⁴, n, p und q die oben angegebenen Bedeutungen haben, oder
- R² und R³ zusammen mit dem Stickstoffatom, an das sie gebunden sind, eine heterocyclische Gruppe, ausgewählt aus Piperidyl-, Pyrrolidinyl-, Morpholinyl-, Thiomorpholinyl-, Tetrahydropyridyl-, Imidazolidinyl-, Indolinyl-, Tetrahydrochinolyl- und Perhydroindolylgruppen, bilden, und
- m steht für eine ganze Zahl von 6 bis 8.

2. Calixaren-Derivat nach Anspruch 1, in dem R¹ für eine Alkoxy- oder Arylalkoxygruppe steht.

3. Calixaren-Derivat nach Anspruch 2, in dem die Alkoxygruppe die Pentoxy- oder Octoxygruppe darstellt.

4. Calixaren-Derivat nach Anspruch 2, in dem die Arylalkoxygruppe die Benzyloxygruppe darstellt.

5. Calixaren-Derivat nach einem der Ansprüche 1 bis 4, in dem R² und R³ Alkylgruppen darstellen.

6. Calixaren-Derivat nach einem der Ansprüche 1 bis 5, in dem m für die Zahl 6 oder 8 steht.

7. Calixaren-Derivat, ausgewählt aus der Gruppe:
- 5,11,17,23,29,35,41,47-Octabenzyloxy-49,50,51,52,53,54,55,56-octakis-[(N,N-diethylamino-carbonyl)methoxy]calix[8]aren,
- 5,11,17,23,29,35,41,47-Octamethoxy-49,50,51,52,53,54,55,56-octakis[(N,N-diethylamino-carbonyl)methoxy]calix[8]aren,
- 5,11,17,23,29,35-Hexamethoxy-37,38,39,40,41,42-hexakis-[(N,N-diethylaminocarbonyl)methoxy]-calix[6]aren,
- 5,11,17,23,29,35-Hexabenzyloxy-37,38,39,40,41,42-hexakis-[(N,N-diethylamino-carbonyl)methoxy]calix[6]aren,
- 5,11,17,23,29,35,41,47-Octakis(1,1-dimethylethyl)-49,50,51,52,53,54,-55,56-octakis[N,N-diethylamino-carbonyl)methoxy]calix[8]aren,
- 49,50,51,52,53,54,55,56-Octakis[N,N-diethylamino-carbonyl)methoxy]-calix[8]aren,
- 5,11,17,23,29,35,41,47-Octapentoxy-49,50,51,52,53,54,55,56-octakis[(N,N-diethylamino-carbonyl)methoxy]calix[8]aren,-
- 5,11,17,23,29,35,41,47-Octaoctoxy-49,50,51,52,53,54,55,56-octakis-[N,N-diethylamino-carbonyl)methoxy]calix[8]aren,
- 5,11,17,23,29,35,41,47-Octahydroxy-49,50,51,52,53,54,55,56-octakis[(N, N-diethylamino-carbonyl)methoxy]calix[8]aren,
- 5,11,17,23,29,35,41-Heptakis-(1,1-dimethylethyl)-43,44,45,46,47,48,49-heptakis-(N,N-diethylamino-carbonyl)methoxy]calix[8]aren und
- 5,11,17,23,29,35-Hexahydroxy-37,38,39,40,41,42-hexakis-[(N,N-diethylaminocarbonyl)methoxy]calix[6]aren.

8. Verfahren zur Herstellung eines Calixaren-Derivats der Formel: 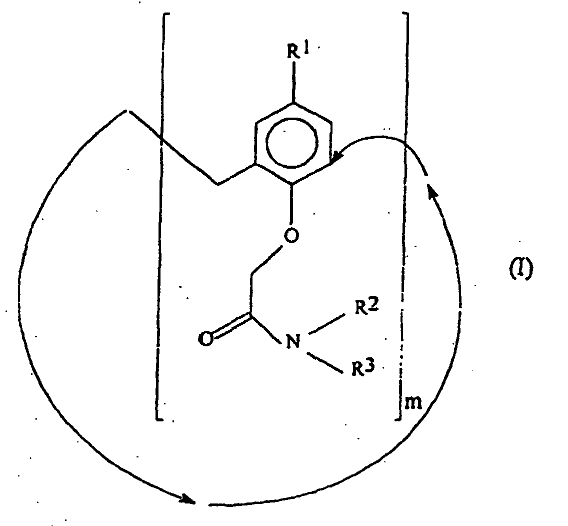 worin bedeuten:
- R¹ ein Wasserstoffatom oder eine Benzyloxygruppe,
- R² und R³, die gleich oder verschieden sein können, eine Alkyl-, Cycloalkyl- oder Arylgruppe oder eine Gruppe der Formel: O(CH₂)ₙ[O(CH₂)ₚ]_{q}OR⁴ (II), in der R⁴, n, p und q die oben angegebenen Bedeutungen haben, oder
- R² und R³ zusammen mit dem Stickstoffatom, an das sie gebunden sind, eine heterocyclische Gruppe, ausgewählt aus Piperidyl-, Pyrrolidinyl-, Morpholinyl-, Thiomorpholinyl-, Tetrahydropyridyl-, Imidazolidinyl-, Indolinyl-, Tetrahydrochinolyl- und Perhydroindolylgruppen, bilden und
- m steht für eine ganze Zahl von 6 bis 8,
wobei das Verfahren umfasst die Umsetzung eines Calixarens der Formel: 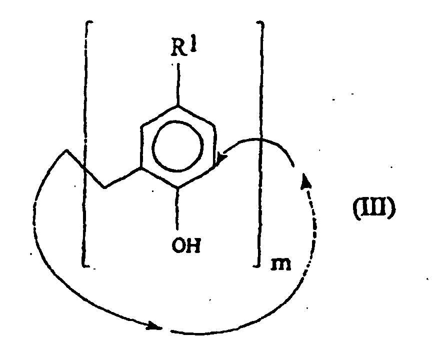 in der R¹ und m die oben angegebenen Bedeutungen haben,
mit einem Chloracetamid der Formel: 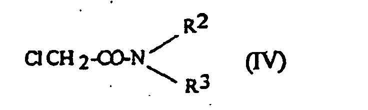 in der R² und R³ die oben angegebenen Bedeutungen haben.

9. Verfahren zur Herstellung eines Calixaren-Derivats der Formel: 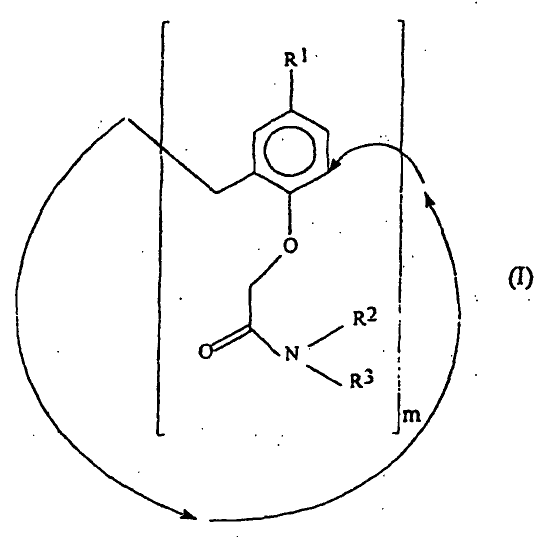 worin bedeuten:
- R¹ eine Alkoxygruppe,
- R² und R³, die gleich oder verschieden sein können, eine Alkyl-, Cycloalkyl- oder Arylgruppe oder eine Gruppe der Formel O(CH₂)ₙ[O(CH₂)ₚ]_{q}OR⁴ (II), in der R⁴, n, p und q die oben angegebenen Bedeutungen haben, oder
- R² und R³ zusammen mit dem Stickstoffatom, an das sie gebunden sind, eine heterocyclische Gruppe, ausgewählt aus Piperidyl-, Pyrrolidinyl-, Morpholinyl-, Thiomorpholinyl-, Tetrahydropyridyl-, Imidazolidinyl-, Indolinyl-, Tetrahydrochinolyl- und Perhydroindolylgruppen, bilden und
- m steht für eine ganze Zahl von 6 bis 8,
wobei das Verfahren die folgenden Stufen umfasst:
a) Umsetzung eines Calixarens der Formel: 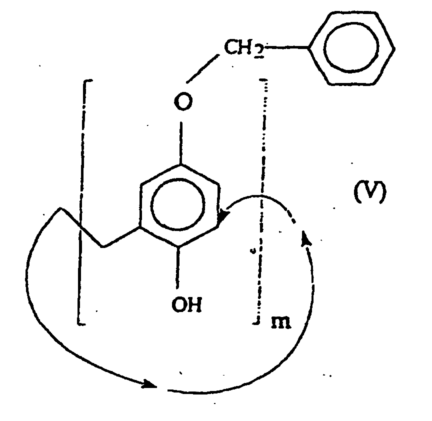 in der m wie oben definiert ist,
mit einem Chloracetamid der Formel: 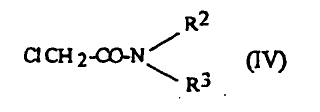 in der R² und R³ wie oben definiert sind,
zur Herstellung des Derivats der Formel 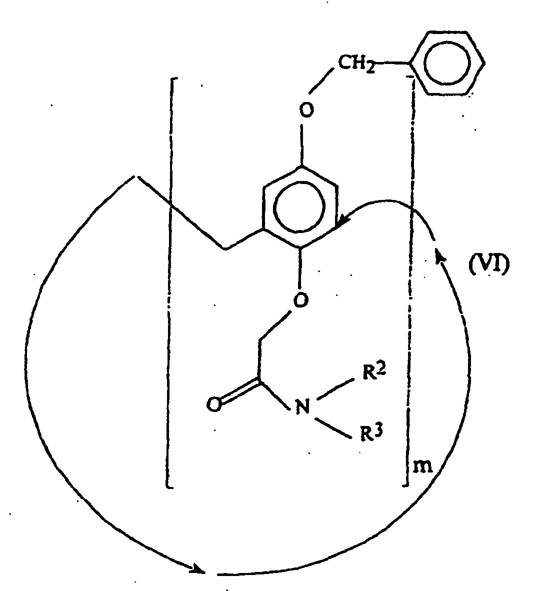 in der R² und R³ wie oben definiert sind,
b) Umsetzung des Derivats der Formel (VI) mit Pd(OH)₂ zur Herstellung des Calixarens der Formel: 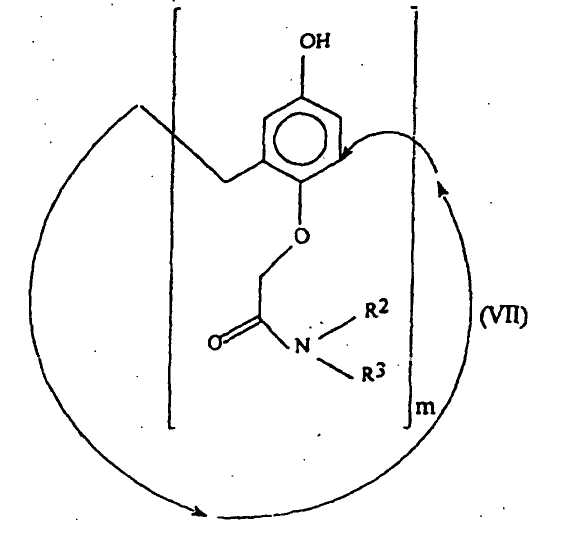
c) Umsetzung des Calixarens der Formel (VII) mit einem halogenierten Derivat der Formel R⁵X, in der R⁵ für eine Alkylgruppe und X für ein Halogenatom stehen, zur Herstellung des Calixaren-Derivats der Formel (I), in der R¹ die Alkoxygruppe OR⁵ darstellt.

10. Verfahren zur Herstellung eines Calixaren-Derivats der Formel: 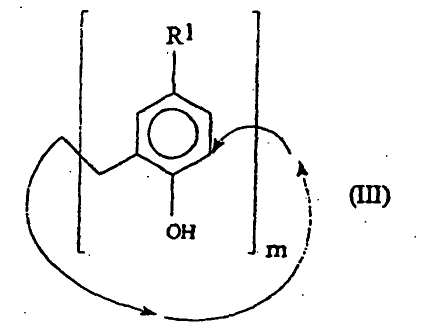 in der R¹ die Benzyloxygruppe und m die Zahl 6 oder 8 bedeuten, durch Durchführung einer Kondensationsreaktion mit p-Benzyloxyphenol der Formel: 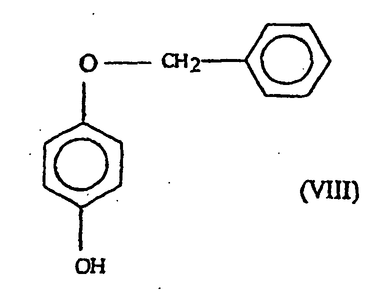 zur Herstellung einer Mischung von Calix[6]aren, Calix[7]aren und Calix[8]aren der Formel (III) und
Abtrennung des Calixares der Formel (III) mit m = 6 oder 8.

11. Verfahren zum Extrahieren des in einer wässrigen Lösung vorhandenen Strontiums, das darin besteht, dass man die wässrige Lösung mit einer damit nicht mischbaren Phase in Kontakt bringt, die mindestens ein Calixaren-Derivat der Formel enthält: 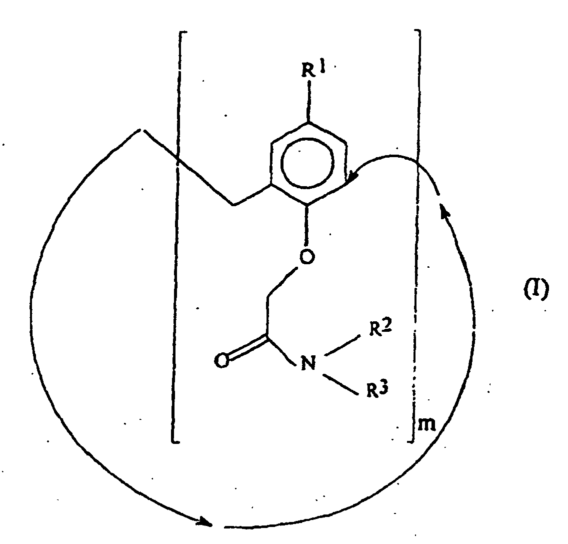 worin bedeuten:
- R¹ ein Wasserstoffatom, eine Alkyl-, Alkoxy-, Aryl-, Aryloxy-, Arylalkyl-, Arylalkoxy-, Cycloalkyl- oder Cycloalkoxy-Gruppe oder eine Gruppe der Formel O(CH₂)ₙ[O(CH₂)ₚ]_{q}OR⁴ (II), in der R⁴ für ein Wasserstoffatom oder eine Alkylgruppe steht, n und p ganze Zahlen von 1 bis 6 darstellen und q für die Zahl 0 oder eine ganze Zahl von 1 bis 6 steht,
- R² und R³, die gleich oder verschieden sein können, eine Alkyl-, Cycloalkyl- oder Arylgruppe oder eine Gruppe der Formel O(CH₂)ₙ[O(CH₂)ₚ]_{q}OR⁴ (II), in der R⁴, n, p und q die oben angegebenen Bedeutungen haben, oder
- R² und R³ zusammen mit dem Stickstoffatom, an das sie gebunden sind, eine heterocyclische Gruppe, ausgewählt aus Piperidyl-, Pyrrolidinyl-, Morpholinyl-, Thiomorpholinyl-, Tetrahydropyridyl-, Imidazolidinyl-, Indolinyl-, Tetrahydrochinolyl- und Perhydroindolylgruppen, bilden und
- m steht für eine ganze Zahl von 6 bis 8,
um das Strontium in die nicht mischbare Phase zu extrahieren.

12. Verfahren nach Anspruch 11, in dem die wässrige Lösung eine Salpetersäure-Lösung mit 1 bis 4 mol/l Salpetersäure oder eine Kochsalzlösung, die Natriumnitrat in einer Konzentration von 1 bis 4 mol/l enthält, ist.

13. Verfahren nach Anspruch 11, bei dem die nicht mischbare Phase eine Lösung des Calixaren-Derivats der Formel (I) in einem organischen Lösungsmittel ist.

14. Verfahren nach Anspruch 13, in dem das organische Lösungsmittel ein Nitrophenylalkylether ist.

## Claims

1. Derivative of calixarene having the formula: 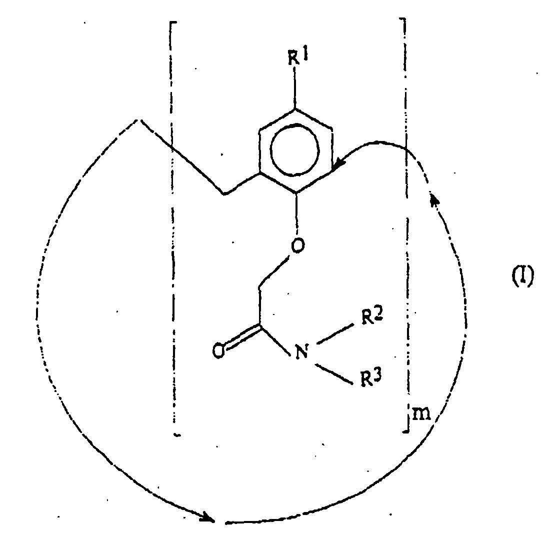 in which:
- R¹ represents a hydroxyl, alcoxy, aryloxy, arylalcoxy, or cycloalcoxy group, or a group having the formula O(CH₂)ₙ [O(CH₂)ₚ]_{q} OR⁴ (II) in which R⁴ represents a hydrogen atom or an alkyl group, n and p are whole numbers ranging from 1 to 6, and q equals 0 or is a whole number from 1 to 6,
- R² and R³, which may identical or different, represent an alkyl, cycloalkyl or aryl group or a group having the formula: O(CH₂)ₙ [O(CH₂)ₚ]_{q} OR⁴ (II) in which R⁴, n, p and q are such as defined above, or
- R² and R³, together with the nitrogen atom to which they are bound, form a heterocyclic group chosen from among the piperidyl, pyrrolidinyl, morpholinyl, thiomorpholinyl, tetrahydropyridyl, imidazolidinyl, indolinyl, tetrahydroquinolyl and perhydroindolyl groups, and
- m is a whole number ranging from 6 to 8.

2. Calixarene derivative according to claim 1, in which R¹ is an alcoxy or arylalcoxy group.

3. Calixarene derivative according to claim 2, in which the alcoxy group is the pentoxy or octoxy group.

4. Calixarene derivative according to claim 2, in which the arylalcoxy group is the benzyloxy group.

5. Calixarene derivative according to any of claims 1 to 4, in which R² and R³ are alkyl groups.

6. Calixarene derivative according to any of claims 1 to 5, in which m equals 6 or 8.

7. Calixarene derivative chosen from among:
5,11,17,23,29,35,41,47-octabenzyloxy-49,50,51,52,53,54,55,56-octakis [N,N-diethylaminocarbonyl)methoxy]calix[8]arene,
5,11,17,23,29,35,41,47-octamethoxy-49,50,51,52,53,54,55,56-octakis [N,N-diethylaminocarbonyl)methoxy]calix[8]arene,
5,11,17,23,29,35-hexamethoxy-37,38,39,40,41,42-hexakis [N,N-diethylaminocarbonyl)methoxy]-calix[6] arene,
5,11,17,23,29,35-hexabenzyloxy-37,38,39,40,41,42-hexakis [N,N-diethylamino-carbonyl)methoxy]calix[6] arene,
5,11,17,23,29,35,41,47-octakis(1,1-dimethylethyl)-49,50,51,52,53,54,55,56-octakis [N,N-diethylaminocarbonyl)methoxy]calix[8]arene,
49,50,51,52,53,54,55,56-octakis [N,N-diethylaminocarbonyl)methoxy]calix[8]arene,
5,11,17,23,29,35,41,47-octapentoxy-49,50,51,52,53,54,55,56-octakis [N,N-diethylaminocarbonyl)methoxy]calix[8]arene,
5,11,17,23,29,35,41,47-octaoctoxy-49,50,51,52,53,54,55,56-octakis [N,N-diethylaminocarbonyl)methoxy]calix[8]arene,
5,11,17,23,29,35,41,47-octahydroxy-49,50,51,52,53,54,55,56-octakis [N,N-diethylaminocarbonyl)methoxy]calix[8]arene,
5,11,17,23,29,35,41-heptakis (1,1-dimethylethyl)-43,44,45,46,47,48,49-heptakis (N,N-diethylaminocarbonyl)methoxy]calix[8]arene, and
5,11,17,23,29,35-hexahydroxy-37,38,39,40,41,42-hexakis [N,N-diethylaminocarbonyl)methoxy]calix(6] arene.

8. Method for preparing a calixarene derivative having the formula: 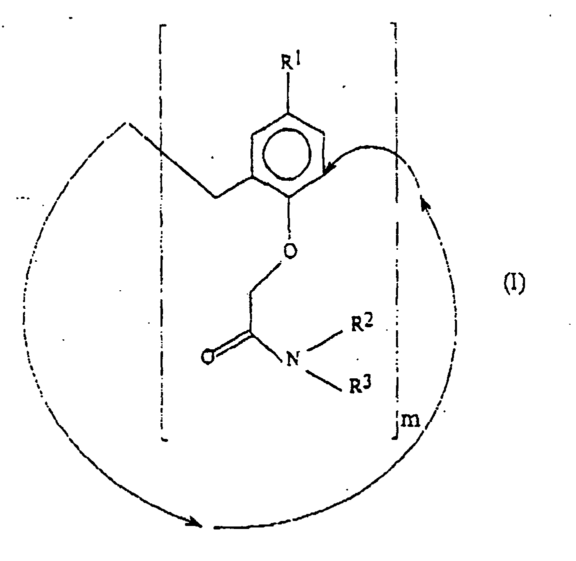 in which:
- R¹ represents a hydrogen atom, or a benzyloxy group,
- R² and R³, which may identical or different, represent an alkyl, cycloalkyl or aryl group or a group having the formula: O(CH₂)ₙ [O(CH₂)ₚ]_{q} OR⁴ (II) in which R⁴, n, p and q are such as defined above, or
- R² and R³, together with the nitrogen atom to which they are bound, form a heterocyclic group chosen from among the piperidyl, pyrrolidinyl, morpholinyl, thiomorpholinyl, tetrahydropyridyl, imidazolidinyl, indolinyl, tetrahydroquinolyl and perhydroindolyl groups, and
- m is a whole number ranging from 6 to 8,
which comprises the reaction of a calixarene having the formula: 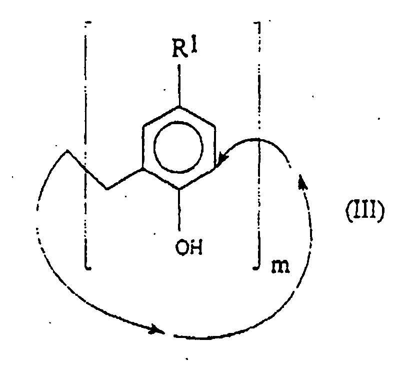 in which R¹ and m are such as defined above, with a chloroacetamide having the formula: 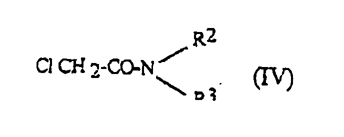 in which R² and R³ are such as defined above.

9. Method for preparing a calixarene derivative having the formula: 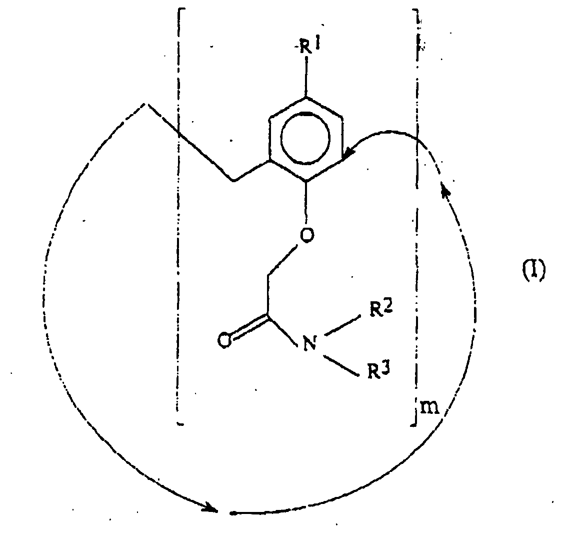 in which:
- R¹ represents an alcoxy group,
- R² and R³, which may identical or different, represent an alkyl, cycloalkyl or aryl group or a group having the formula: O(CH₂)ₙ [O(CH₂)ₚ]_{q} OR⁴ (II) in which R⁴, n, p and q are such as defined above, or
- R² and R³, together with the nitrogen atom to which they are bound, form a heterocyclic group chosen from among the piperidyl, pyrrolidinyl, morpholinyl, thiomorpholinyl, tetrahydropyridyl, imidazolidinyl, indolinyl, tetrahydroquinolyl and perhydroindolyl groups, and
- m is a whole number ranging from 6 to 8,
which comprises the following steps:
a) reaction of a calixarene having the formula: 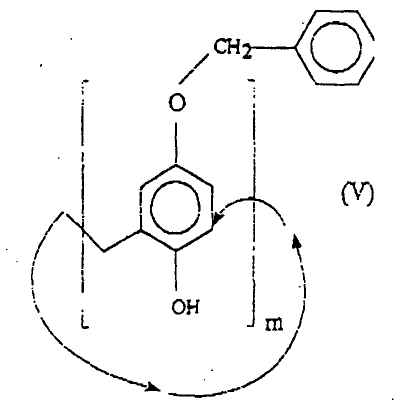 in which m is such as defined above, with a chloroacetamide having the formula: 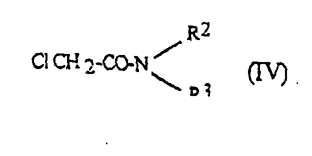 in which R² and R³ are such as defined above, in order to obtain the derivative having the formula: 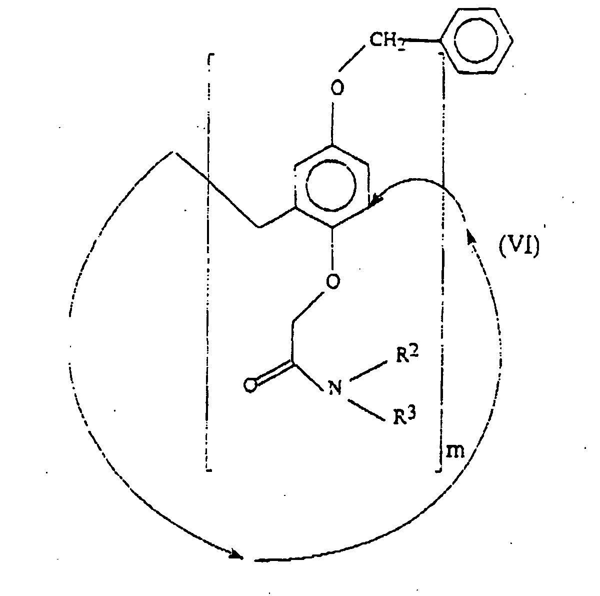 in which R² and R³ are such as defined above,
b) reaction of the derivative of formula (VI) with Pd(OH₂) to obtain the calixarene having the formula: 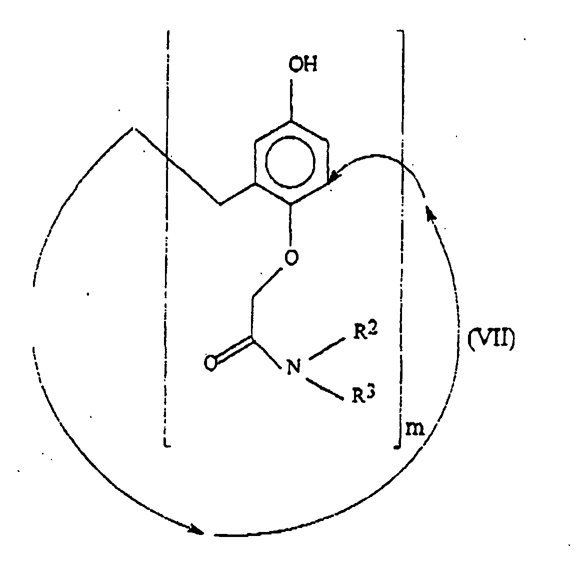
c) reaction of the calixarene of formula VII with a halogenated derivative of formula R⁵X in which R⁵ represents an alkyl group and X is a halogen atom, to obtain the calixarene derivative of formula (I) in which R¹ represents the alcoxy group OR⁵.

10. Method for preparing a., calixarene derivative having the formula: 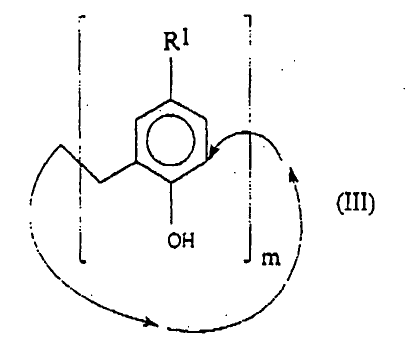 in which R¹ represents the benzyloxy group and m equals 6 or 8, by condensation reaction of p-benzyloxyphenol having the formula: 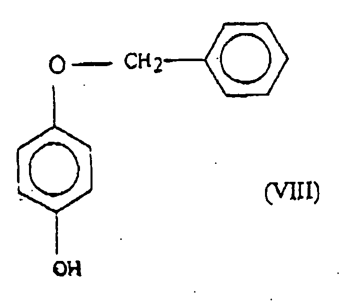 to obtain a mixture of calix[6]arene, calix[7]arene and calix[8]arene of formula (III), and separation of the calixarene of formula (III) in which m = 6 or 8.

11. Method for extracting strontium present in an aqueous solution, which consists of contacting the aqueous solution with an immiscible phase containing at least one calixarene derivative having the formula: 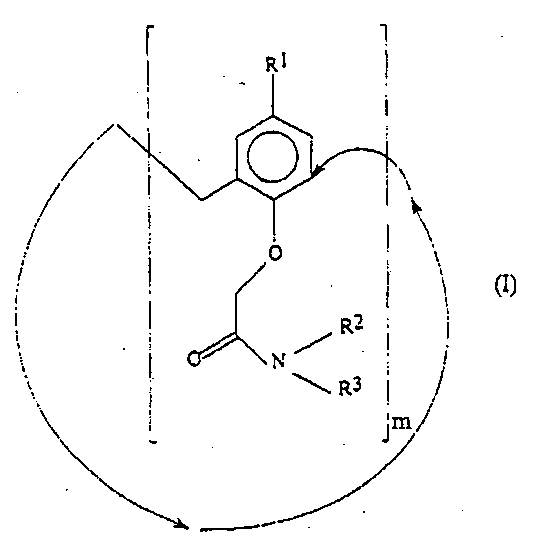 in which:
- R¹ represents a hydrogen atom, an alkyl, alcoxy, aryl, aryloxy, arylalkyl, arylalcoxy, cycloalkyl or cycloalcoxy group, or a group having the formula: O(CH₂)ₙ [O(CH₂)ₚ]_{q} OR⁴ (II) in which R⁴ represents a hydrogen atom or an alkyl group, n and p are whole numbers ranging from 1 to 6, and q equals 0 or is a whole number from 1 to 6,
- R² and R³, which may identical or different, represent an alkyl, cycloalkyl or aryl group or a group having the formula: O(CH₂)ₙ [O(CH₂)ₚ]_{q} OR⁴ (II) in which R⁴, n, p and q are such as defined above, or
- R² and R³, together with the nitrogen atom to which they are bound, form a heterocyclic group chosen from among the piperidyl, pyrrolidinyl, morpholinyl, thiomorpholinyl, tetrahydropyridyl, imidazolidinyl, indolinyl, tetrahydroquinolyl and perhydroindolyl groups, and
- m is a whole number ranging from 6 to 8,
to extract strontium in the immiscible phase.

12. Method according to claim 11, in which the aqueous solution is a nitric solution containing from 1 to 4 mol/l nitric acid, or a saline solution having a sodium nitrate concentration of 1 to 4 mol/L.

13. Method according to claim 11 or 12, in which the immiscible phase is a solution of the derivative of calixarene of formula (I) in an organic solvent.

14. Method according to claim 13, in which the organic solvent is a nitrophenyl-alkylether.
